# EUROPEAN PATENT SPECIFICATION

(11) **EP 3 080 157 B1**
(45) Date of publication and mention of the grant of the patent: **03.10.2018**
(21) Application number: 14827185.1
(22) Date of filing: 15.12.2014
(51) Int. Cl.: A61K 38/00, A61L 26/00, C07K 14/81

(54) **MODIFIED SERPINS FOR THE TREATMENT OF BLEEDING DISORDERS**
MODIFIZIERTE SERPINE ZUR BEHANDLUNG VON BLUTUNGSSTÖRUNGEN
SERPINES MODIFIÉES POUR LE TRAITEMENT DE TROUBLES DE SAIGNEMENT

(30) Priority: 13.12.2013 GB 201322091
(43) Date of publication of application: 19.10.2016
(60) Divisional application: 18190334.5
(73) Proprietor: Cambridge Enterprise Limited, Cambridge, Cambridgeshire CB2 1TN (GB)
(72) Inventor: HUNTINGTON, James Andrew, Cambridge Cambridgeshire CB2 0XY (GB); POLDERDIJK, Stéphanie, Cambridge Cambridgeshire CB2 0XY (GB); BAGLIN, Trevor, Cambridge Cambridgeshire CB2 0XY (GB)
(74) Representative: Sutcliffe, Nicholas Robert
(86) International application number: PCT/EP2014/077783
(87) International publication number: WO 2015/086854

(56) References cited:
- WO-A2-2006/090282
- WO-A2-2008/045148
- M. I. PLOTNICK ET AL: "The Effects of Reactive Site Location on the Inhibitory Properties of the Serpin alpha 1-Antichymotrypsin", JOURNAL OF BIOLOGICAL CHEMISTRY, vol. 277, no. 33, 7 June 2002 (2002-06-07) , pages 29927-29935, XP055172584, ISSN: 0021-9258, DOI: 10.1074/jbc.M202374200
- MARC L. FILION ET AL: "Full or Partial Substitution of the Reactive Center Loop of [alpha]-1-Proteinase Inhibitor by that of Heparin Cofactor II: P1 Arg Is Required for Maximal Thrombin Inhibition", BIOCHEMISTRY, vol. 43, no. 46, 1 November 2004 (2004-11-01), pages 14864-14872, XP055172656, ISSN: 0006-2960, DOI: 10.1021/bi048833f
- HWANG ET AL: "Multiple domains of endopin 2A for serpin cross-class inhibition of papain", ARCHIVES OF BIOCHEMISTRY AND BIOPHYSICS, ACADEMIC PRESS, US, vol. 461, no. 2, 5 May 2007 (2007-05-05), pages 219-224, XP022065136, ISSN: 0003-9861, DOI: 10.1016/J.ABB.2007.02.036

## Description

### Field

This invention relates to modified serpin molecules with altered specificity, in particular serpin molecules modified to have increased specificity for anticoagulant proteases, such as activated Protein C (APC).

### Background

Hemophilias are bleeding disorders, which are caused by a deficiency in circulating plasma fVIII (hemophilia A, HA) or fIX (hemophilia B, HB) (reviewed in Bolton-Maggs & Pasi, 2003). This reduces the activity of the intrinsic tenase (Xase) and thereby the amount of thrombin generated when tissue injury occurs. This leads to uncontrolled bleeding after injury as well as spontaneous bleeding into joints and soft tissue.

Hemophilia affects around 1 in 5,000 people. The 170,000 patients identified in the World Federation of Hemophilia Global Survey is an underestimate of the global health burden (World Federation of Hemophilia, 2011). The treatment costs are very high and treatment is frequent and lifelong.

Standard treatments for hemophilia entail replacement of the clotting factor affected, using either recombinant or plasma-derived factors (reviewed in Mannucci, 2003; 2008). However, a significant proportion of patients treated in this manner will develop inhibitory antibodies against the supplemented coagulation factor, rendering the treatment ineffective (reviewed in Brettler, 1996). Inhibitors occur in 30% of treated patients with hemophilia (reviewed in Teitel & Sholzberg 2013) but global estimates are low due to high mortality in untreated inhibitor patients and a low prevalence of inhibitors in many countries in which factor VIII replacement therapy is not available. Another drawback of conventional therapies is their expense, as well as the short half-life of the injected clotting factor, necessitating frequent treatments (reviewed in Lee et al, 2006).

In the case where patients develop inhibitory antibodies, bypassing agents are used for treatment of bleeding events (reviewed in (Negrier *et al,* 2006)). Bypassing agents reduce bleeding without directly supplying the clotting factor affected; they 'bypass' the activity of the tenase complex. Examples of current bypassing agents include recombinant fVIIa and FEIBA (Factor Eight_Bypassing Activity), a prothrombin complex concentrate. These replacement treatments are very expensive (Bohn *et al,* 2004; Di Minno *et al,* 2010; Gringeri *et al,* 2003; Escobar, 2010) and need to be given even more frequently than the conventional therapies and in high doses due to the short half-lives of both products (reviewed in Haya *et al,* 2007). In addition, patient response has been shown to be variable and unpredictable (reviewed in Berntorp, 2009).

In addition, the short half-life of factor concentrates renders standard replacement therapy of hemophilia suboptimal. This is particularly evident in hemophilia A as factor VIII has a half-life of less than 12 hours. Consequently, despite the availability of treatment for both hemophilia A and B the bleeding rates are higher in hemophilia A and chronic hemophilic arthropathy is more common. This may be related to the short half-life of factor VIII and consequently the difficulty in maintaining a hemostatic level of factor VIII (Escobar and Sallah 2013). In a national review of treatment the annual frequency of bleeding in patients with severe hemophilia A without inhibitors was 14 compared to 9 in patients with hemophilia B (Nagel, et al 2011). The need for musculoskeletal surgery was 3-times greater in patients with hemophilia A. Tagariello et al found that patients with hemophilia A required joint replacement three times more often than patients with hemophilia B (Tagariello, et al 2009). Lowe et al found that hospitalization was required three times more frequently for patients with hemophilia A compared to hemophilia B (Lowe and Ludlam 2008).

Current treatments for bleeding disorders, such as hemophilia therefore have a range of drawbacks.

WO2008/045148 reports methods for screening modified proteases by contacting them with a substrate, such as a serpin, that, upon cleavage of the substrate, traps the protease by forming a stable complex.

WO2006/090282 reports an hK14 protease inhibitor that comprises the Reactive Serpin Loop of a serpin sequence which is modified by at least one substrate active site sequence specific for the hK14 protease.

### Summary

The present inventors have recognised that the specificity of serpin molecules can be engineered by modification of residues within the reactive center loop (RCL), and have identified modified serpin molecules with increased specificity for anticoagulant proteases. These modified serpin molecules may be useful in therapy, for example for the treatment of bleeding.

An aspect of the invention provides a modified serpin having mutations at residues P1' and P2 and optionally residues P4 and/or P1 in the reactive center loop (RCL) thereof, wherein the P1' residue is mutated to Q, H, K or R; and the P2 residue is mutated to H, K or R, and;
wherein said mutations increase the inhibition of one or more of anticoagulant proteases selected from activated Protein C and thrombin:thrombomodulin complex relative to the inhibition of one or more procoagulant proteases selected from thrombin, fVIIa, fXa, fIXa and fXIa.

The mutations may increase the inhibition of activated Protein C relative to the inhibition of thrombin.

The mutations may also increase the inhibition of activated Protein C relative to the inhibition of other procoagulant proteases, such as fVIIa, fIXa, fXa and fXIa.

Other disclosure relates to the use of modified serpins as described herein for the treatment of bleeding, for example bleeding in patients with heritable bleeding disorders and acquired bleeding, including trauma, surgery and in patients receiving anticoagulant therapy.

### Brief Description of Figures

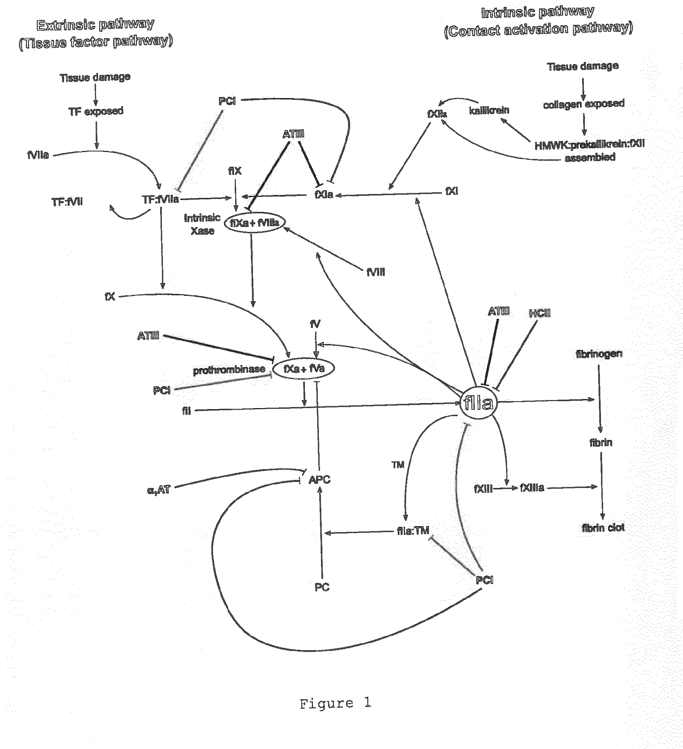

Figure 1 shows the coagulation cascade and the regulatory role of serpins in this cascade.

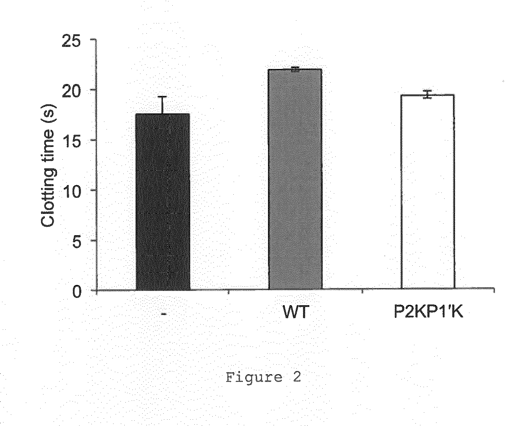

Figure 2 shows the results of a prothrombin time (PT) assay to determine the effect of Protein C Inhibitor (PCI) with a 21 residue N-terminal truncation (N-terminal residue is A22 of the wild-type sequence, when counting residue 1 of the propeptide as residue 1 of the mature protein) and having K residues at the P2 and P1' positions within the RCL (A22 P2KP1'K PCI) on coagulation through the tissue factor pathway (extrinsic). Pooled normal plasma from three separate plasmas was incubated with either no PCI (black bar, -) or 5 µM A22 wild-type (WT) PCI (grey bar, WT) or 5 µM A22 P2KP1'K PCI (white bar, P2KP1'K). Coagulation was initiated by the addition of PT reagent, to initiate coagulation via the extrinsic pathway and the time until clot formation measured. The assay was performed in triplicate, error bars show the standard deviation. 2-fold diluted plasma was used to increase the sensitivity of the assay for inhibitors of coagulation. A22 P2KP1'K PCI has no effect on coagulation in this prothrombin time (PT) assay. This result indicates that there is no significant inhibition of TF: fVIIa, thrombin and other procoagulant proteases by A22 P2KP1'K PCI.

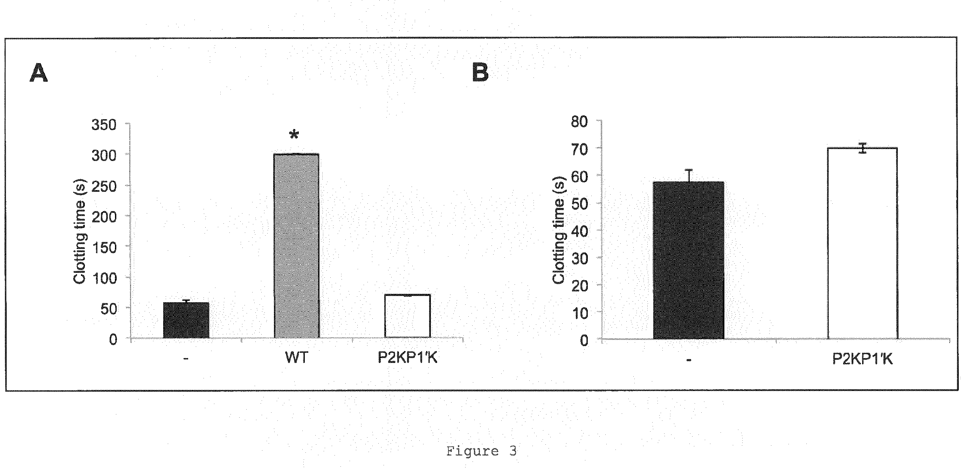

Figure 3 shows the results of an activated partial thromboplastin time (aPTT) assay to determine the effect of A22 P2KP1'K PCI on coagulation through the contact activation pathway (intrinsic). Fig 3A shows pooled normal plasma from three separate plasmas incubated with either no PCI (black bar, -) or 5 µM of A22 wild-type (WT) PCI (grey bar, WT) or A22 P2KP1'K PCI (white bar, P2KP1'K). aPTT reagent was added and the samples incubated for 5 min at 37°C. Coagulation was then initiated by the addition of CaCl₂, to initiate coagulation via the intrinsic pathway and the time until clot formation measured. Bars show averages of at least three measurements, error bars show the standard deviation. The assay was stopped at 300 s. Samples shown at 300 s did not clot within the time of the experiment and are marked with asterisks. Fig 3B shows the data from A without A22 WT PCI samples to show a small effect on the clotting time.

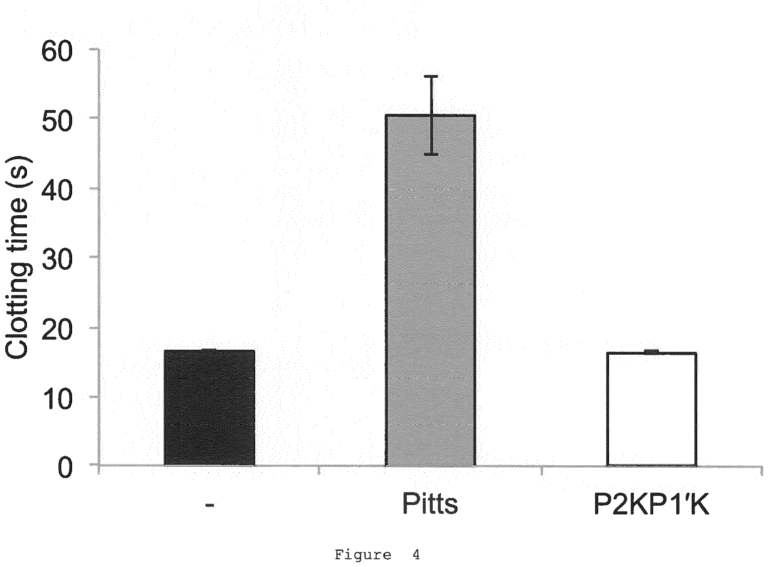

Figure 4 shows the results of a prothrombin time assay (PT) determining the effect of a full-length (FL) α₁-antitrypsin Pittsburgh (Pitts) mutant (M358R = P1R) that further comprises a C232S mutation and P357K (P2) and S359K (P1'K) mutations within the RCL (FL α₁AT Pitts C232S P2KP1'K) on coagulation through the tissue factor pathway (extrinsic). Pooled normal plasma from three separate plasmas was incubated with either no α₁AT (-, black bar) or 5 µM FL α₁AT Pitts C232S (Pitts, grey bar) or 5 µM FL α₁AT Pitts C232S P2KP1'K (P2KP1'K, white bar). Coagulation was initiated by the addition of PT reagent and the time until clot formation measured. Bars show averages of at least three measurements, error bars show the standard deviation. 2-fold diluted plasma was used to increase the sensitivity of the assay for inhibitors of coagulation. FL α₁AT Pitts C232S P2KP1'K, unlike FL α₁AT Pitts C232S does not prolong the PT. This indicates there is no significant inhibitory effect towards any of the procoagulant proteases, including TF-fVIIa, thrombin, or fXa.

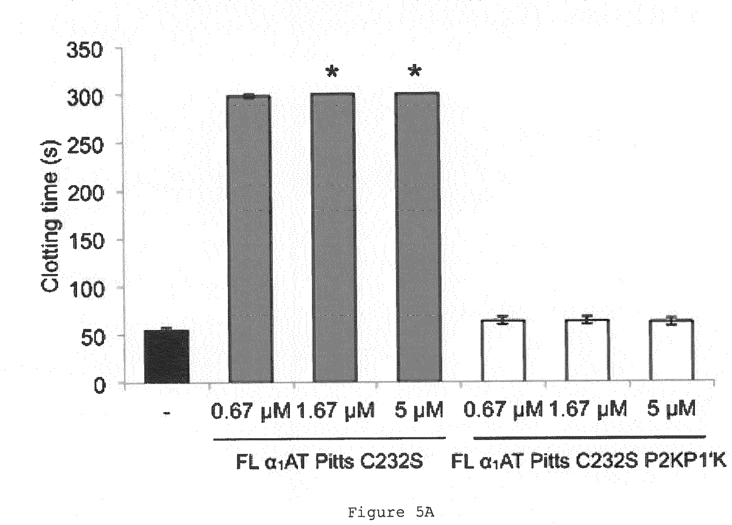

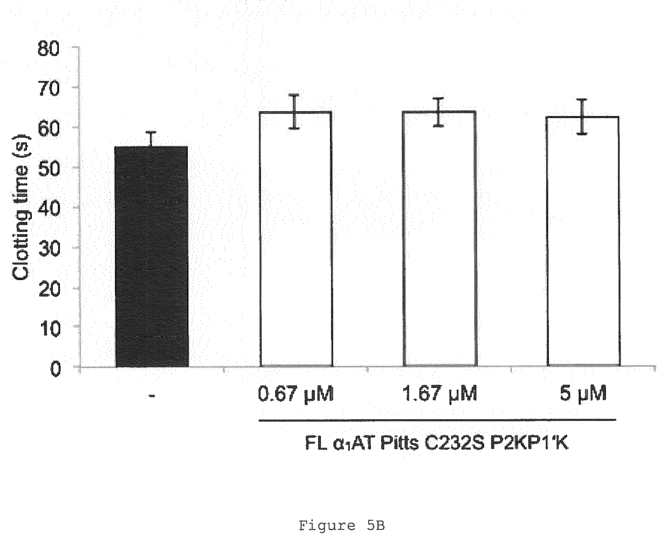

Figure 5 shows the results of an activated partial thromboplastin time assay (aPTT) to determine the effect of FL α₁AT Pitts C232S P2KP1'K on coagulation through the contact activation pathway (intrinsic). Fig 5A shows pooled normal plasma from three separate plasmas incubated with either no α₁AT (black bar) or increasing concentrations of FL α₁AT Pitts C232S (grey bars) or FL α₁AT Pitts C232S P2KP1'K (white bars). aPTT reagent was added and the samples incubated for 5 min at 37°C. Coagulation was then initiated by the addition of CaCl₂ and the time until clot formation measured. Bars show averages of at least three measurements, error bars show the standard deviation. The assay was stopped at 300 s. Samples shown at 300s did not clot within the time of the experiment and are marked with asterisks. Fig 5B shows the data from A without FL α₁AT Pitts C232S samples to show a small effect on the clotting time by FL α₁AT Pitts C232S P2KP1'K. However, this effect does not increase dose-dependently.

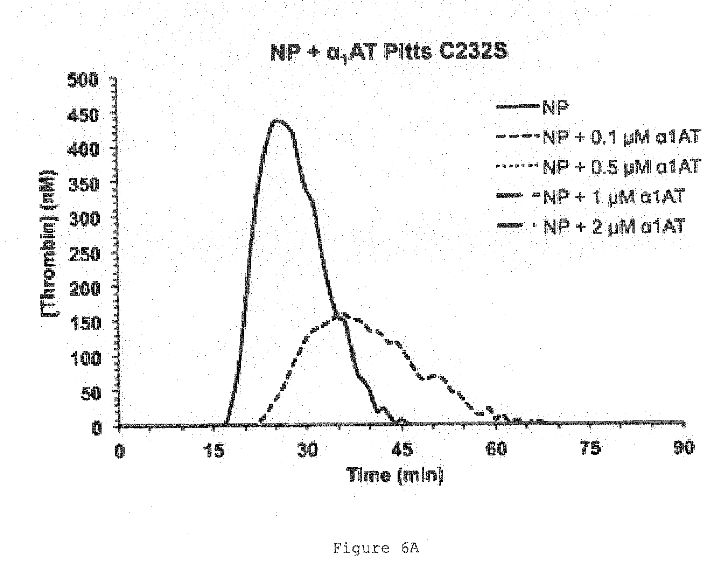

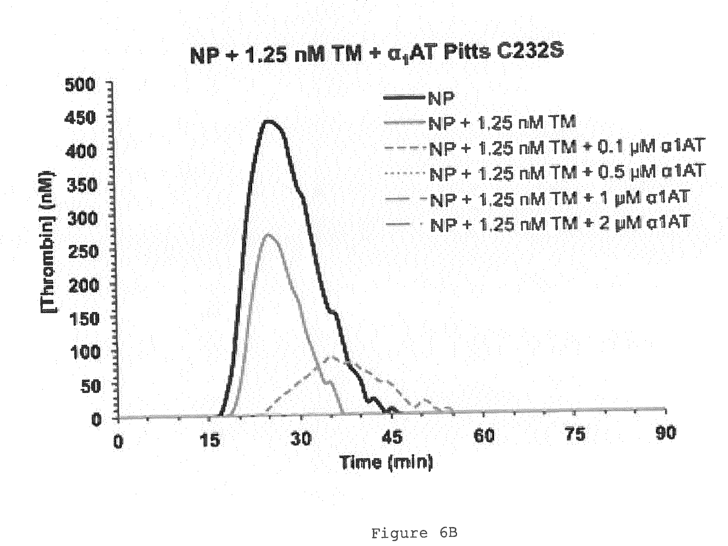

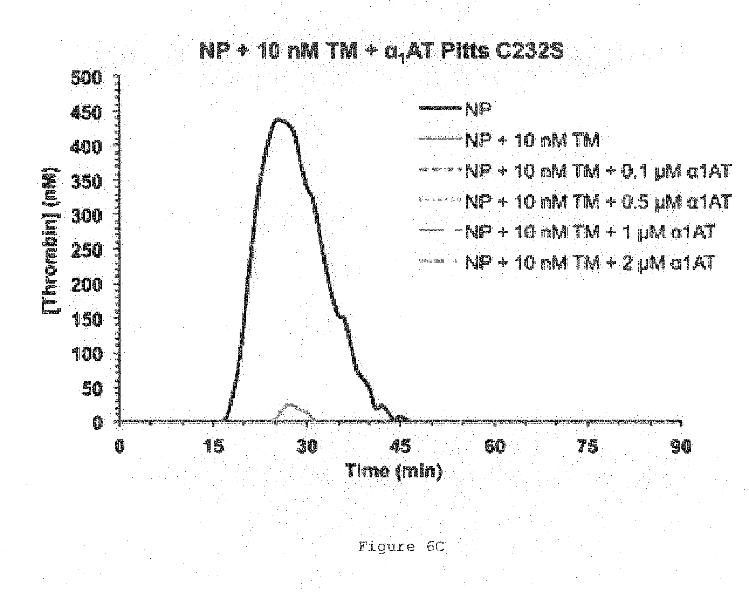

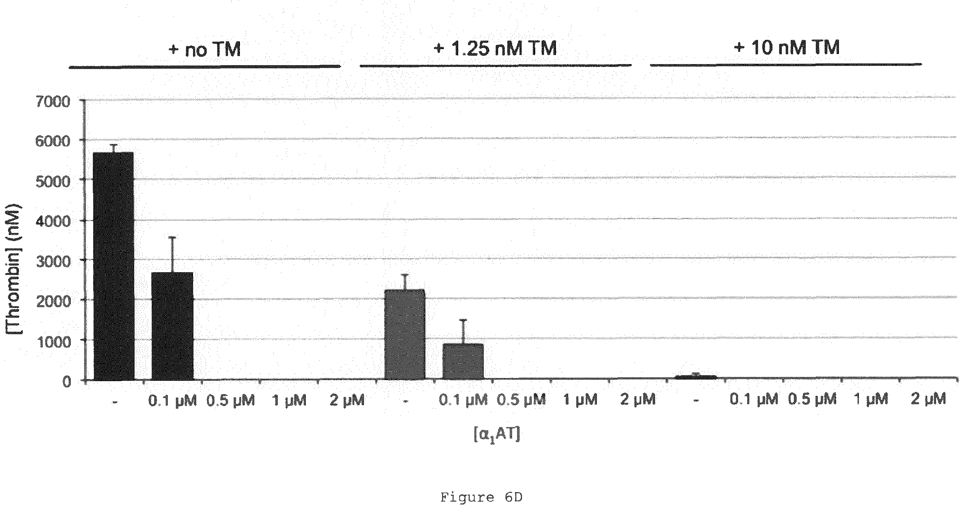

Figure 6 shows that FL α₁AT Pitts C232S inhibits thrombin generation in normal human plasma (NP). Figs A-C show representative thrombin generation curves for reactions containing increasing concentrations of FL α₁AT Pitts C232S in the presence of (A) no thrombomodulin (TM) (B) 1.25 nM thrombomodulin (TM) (C) 10 nM thrombomodulin (TM). Curves show an average of duplicates. All assays were performed in pooled normal human plasma (NP) from George King Biomedical. Coagulation was initiated by the addition of CaCl₂ and TF/phospholipid (RB low TF and phospholipid reagent #5006210, Technoclone GmbH) to activate coagulation through the extrinsic pathway. Thrombin generation was measured through the cleavage of a fluorogenic substrate (Z-Gly-Gly-Arg-AMC). Fluorescence units were converted to thrombin concentration by calibrating fluorescence units against known concentrations of thrombin, using the Technothrombin calibration kit (Technoclone). Fig 6D shows mean ETPs (endogenous thrombin potentials), representing the total amount of thrombin generated during the reactions. Bars show the mean of two independent experiments performed in duplicate. Error bars represent the standard deviation. The thrombomodulin (TM) used was recombinantly produced from HEK-EBNA cells and consists of the extracellular domain of TM.

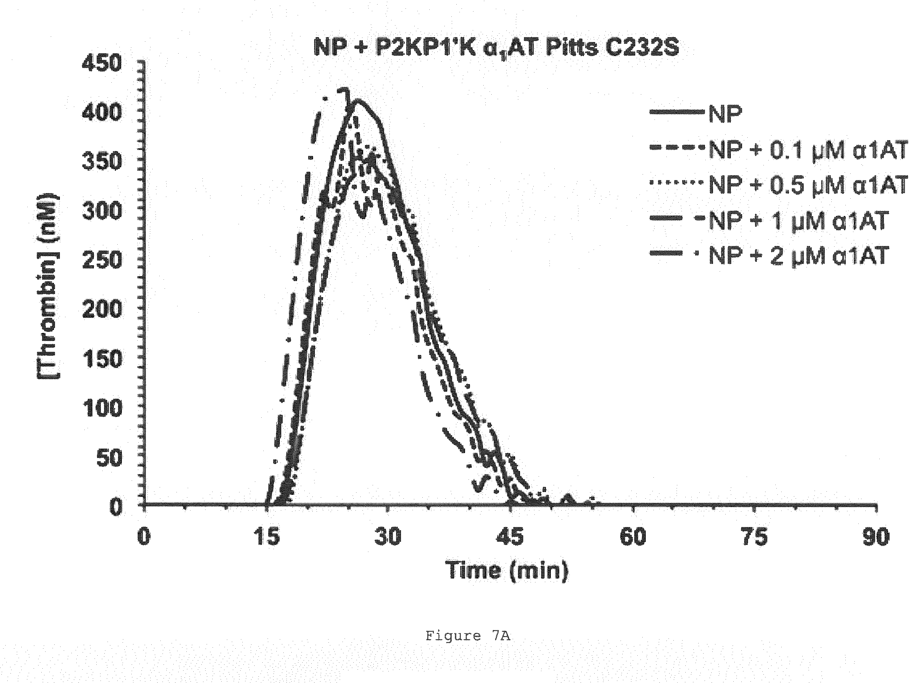

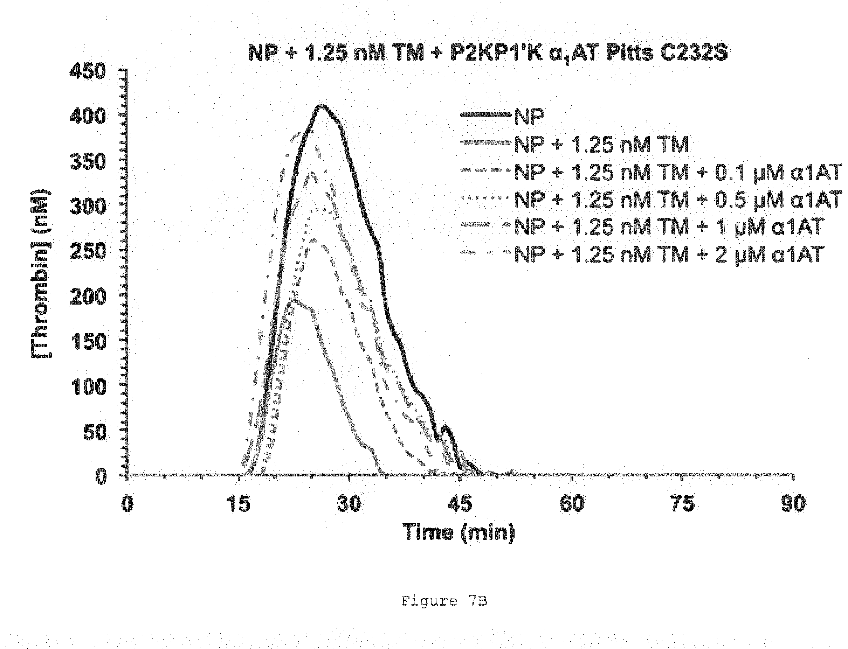

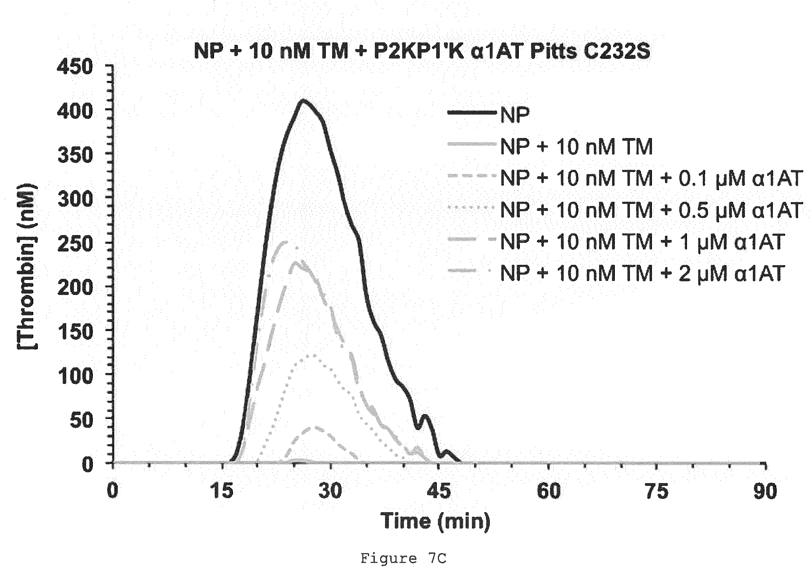

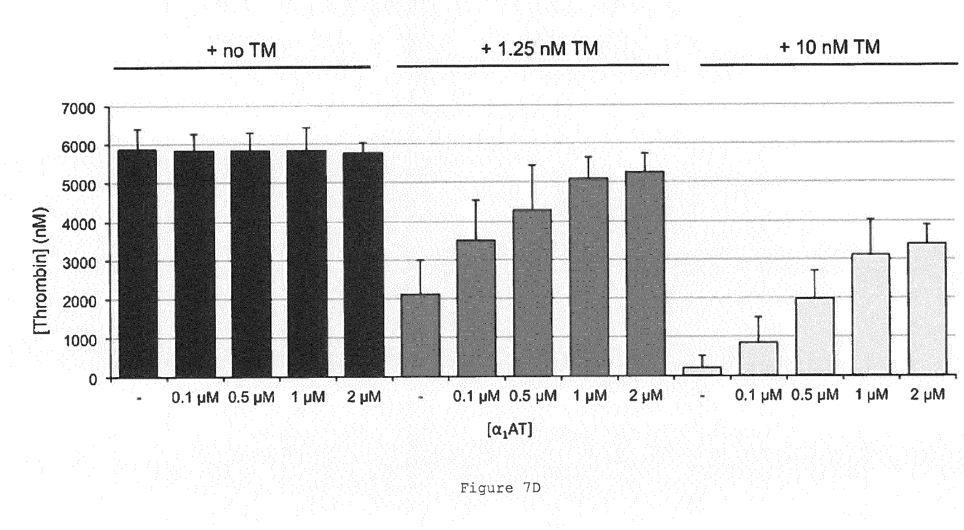

Figure 7 shows that FL α₁AT Pitts C232S P2KP1'K rescues the anticoagulant effect of TM in normal human plasma (NP). Fig 7A-C show representative thrombin generation curves for reactions containing increasing concentrations of FL α₁AT Pitts C232S P2KP1'K in the presence of (A) no TM (B) 1.25 nM TM (C) 10 nM TM. Curves show an average of duplicates. All assays were performed in pooled normal human plasma (NP) from George King Biomedical. Coagulation was initiated by the addition of CaCl₂ and TF/phospholipid (RB low TF and phospholipid reagent #5006210 Technoclone GmbH) to activate coagulation through the extrinsic pathway. Thrombin generation was measured through the cleavage of a fluorogenic substrate (Z-Gly-Gly-Arg-AMC). Fluorescence units were converted to thrombin concentration by calibrating fluorescence units against known concentrations of thrombin, using the Technothrombin calibration kit (Technoclone GmbH). Fig 7D shows mean ETPs (endogenous thrombin potentials), representing the total amount of thrombin generated during the reactions. Bars show the mean of three independent experiments performed in duplicate. Error bars represent the standard deviation.

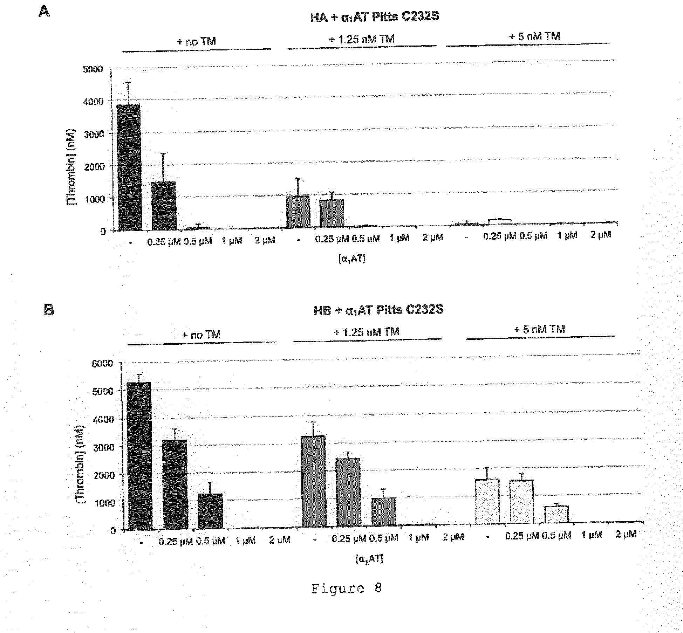

Figure 8 shows that FL α₁AT Pitts C232S abolishes thrombin generation in human hemophilia A plasma (HA, fVIII-deficient), and human hemophilia B plasma (HB, fIX-deficient). Graphs show the mean ETPs from thrombin generation experiments spiked with increasing concentrations of FL α₁AT Pitts C232S in either (A) fVIII-deficient plasma (less than 1% fVIII activity) or (B) fIX-deficient plasma (less than 1% fIX activity) with the indicated amounts of added thrombomodulin (TM). All plasmas were from George King Biomedical. Reactions were initiated by adding CaCl₂ and TF/phospholipid (RB low TF and phospholipid reagent #5006210 Technoclone GmbH) with 1:4,000 final dilution of Dade Innovin (Siemens) to activate coagulation through the extrinsic pathway. Thrombin generation was measured through the cleavage of a fluorogenic substrate (Z-Gly-Gly-Arg-AMC). Fluorescence units were converted to thrombin concentration by calibrating fluorescence units against known concentrations of thrombin, using the Technothrombin calibration kit (Technoclone). Mean ETPs are shown from at least two independent experiments performed in duplicate. Error bars show standard deviations.

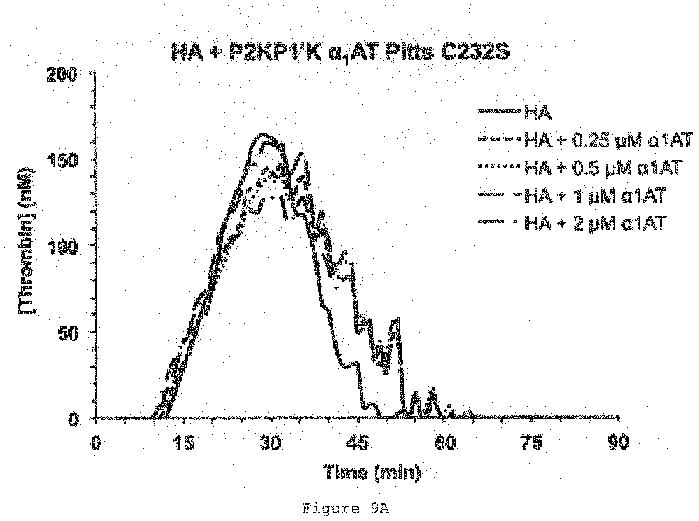

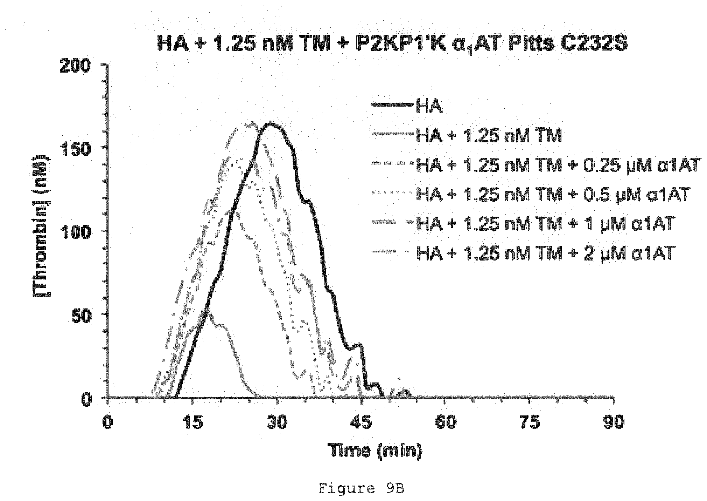

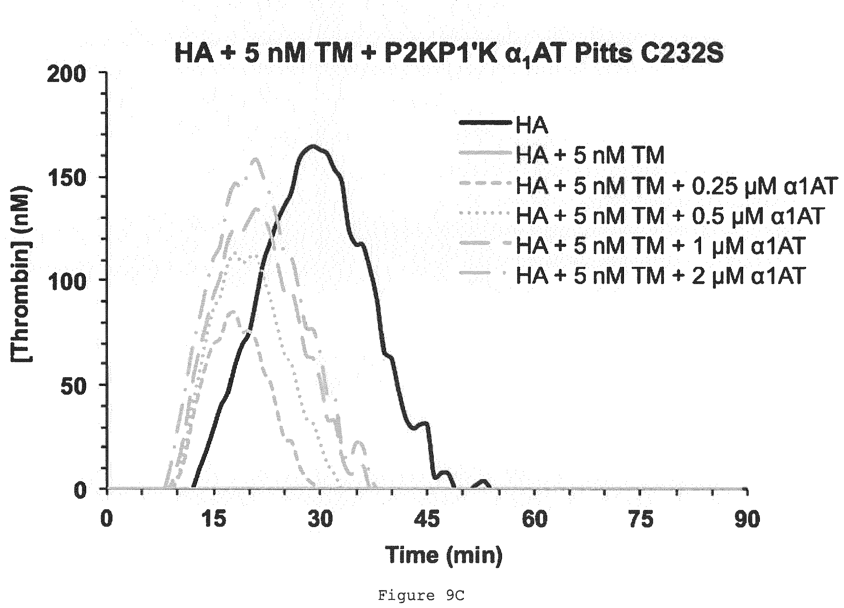

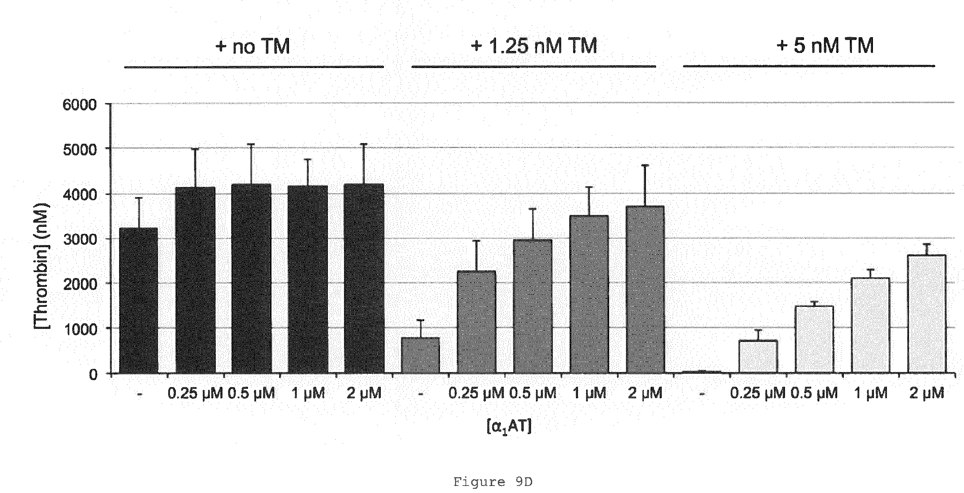

Figure 9 shows that FL α₁AT Pitts C232S P2KP1'K rescues the effect of TM on human HA plasma (fVIII-deficient plasma). Figs 9A-C show representative thrombin generation curves for reactions containing increasing concentrations of FL α₁AT Pitts C232S P2KP1'K in the presence of (A) no TM (B) 1.25 nM TM (C) 5 nM TM. Curves show an average of duplicates. All assays were performed in fVIII-deficient plasma (less than 1% fVIII activity) from George King Biomedical. Coagulation was initiated by the addition of CaCl₂ and TF/phospholipid (RB low TF and phospholipid reagent #5006210, Technoclone GmbH) and 1:4,000 Dade Innovin (Siemens) to activate coagulation through the extrinsic pathway. Thrombin generation was measured through the cleavage of a fluorogenic substrate (Z-Gly-Gly-Arg-AMC). Fluorescence units were converted to thrombin concentration by calibrating fluorescence units against known concentrations of thrombin, using the Technothrombin calibration kit (Technoclone). Fig 9D shows mean ETPs (endogenous thrombin potentials), representing the total amount of thrombin generated during the reactions. Bars show the mean of two independent experiments performed in duplicate. Error bars represent the standard deviation.

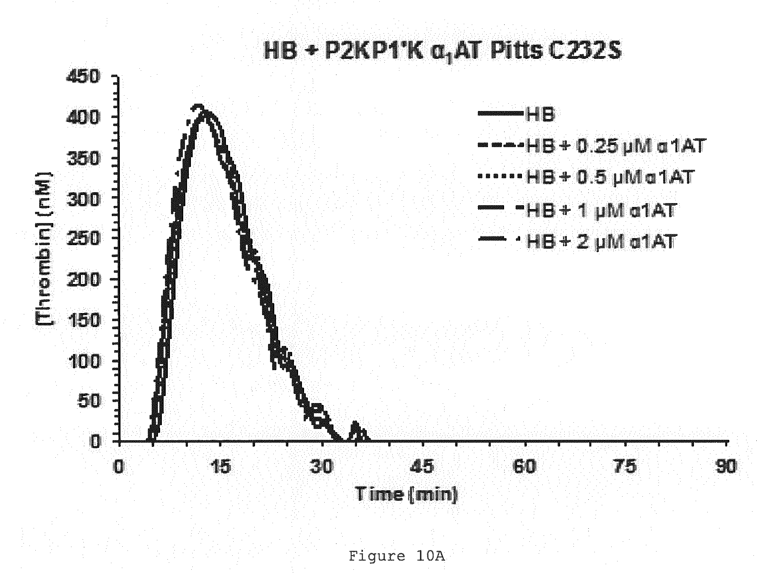

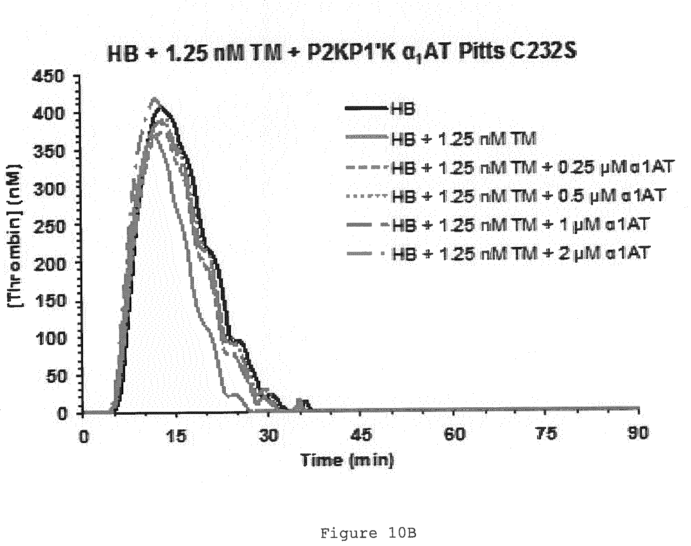

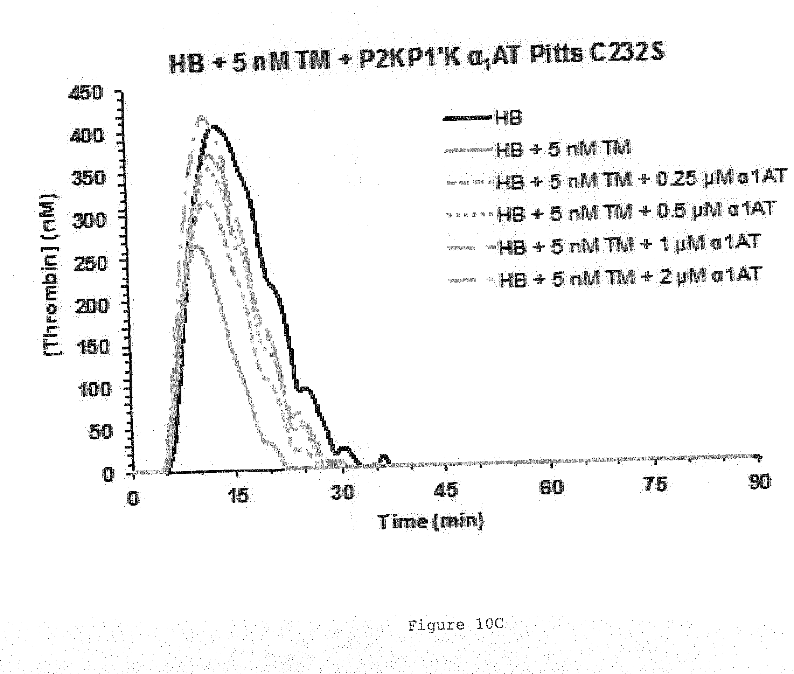

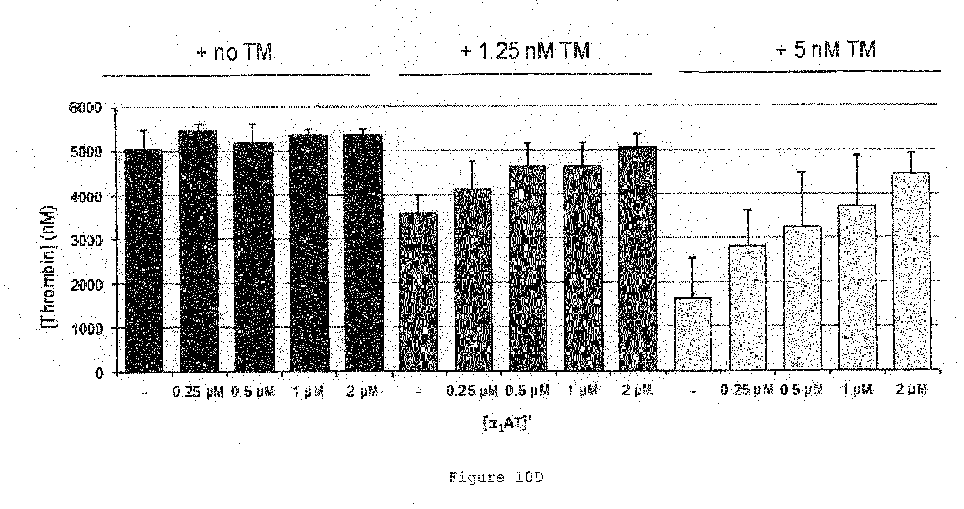

Figure 10 shows that FL α₁AT Pitts C232S P2KP1'K rescues the effect of TM on human HB plasma (fIX-deficient plasma). (A-C) Representative thrombin generation curves are shown for reactions containing increasing concentrations of FL α₁AT Pitts C232S P2KP1'K in the presence of (A) no TM (B) 1.25 nM TM (C) 5 nM TM. Curves show an average of duplicates. All assays were performed in fIX-deficient plasma (less than 1% fIX activity) from George King Biomedical. Coagulation was initiated by the addition of CaCl₂ and TF/phospholipid (RB low TF and phospholipid reagent #5006210, Technoclone GmbH) and 1:4,000 Dade Innovin (Siemens) to activate coagulation through the extrinsic pathway. Thrombin generation was measured through the cleavage of a fluorogenic substrate (Z-Gly-Gly-Arg-AMC). Fluorescence units were converted to thrombin concentration by calibrating fluorescence units against known concentrations of thrombin, using the Technothrombin calibration kit (Technoclone). Fig 10D shows mean ETPs (endogenous thrombin potentials), representing the total amount of thrombin generated during the reactions. Bars show the mean of at least two independent experiments performed in duplicate. Error bars represent the standard deviation.

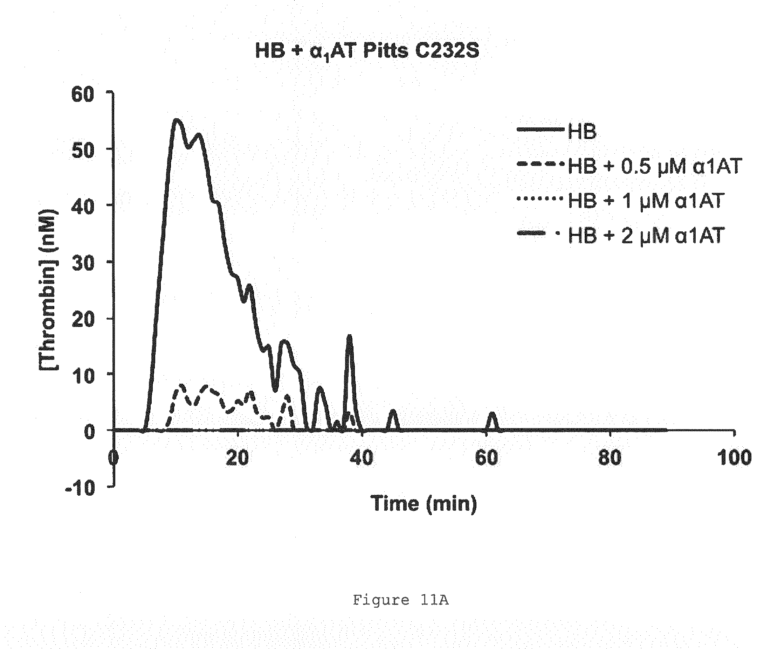

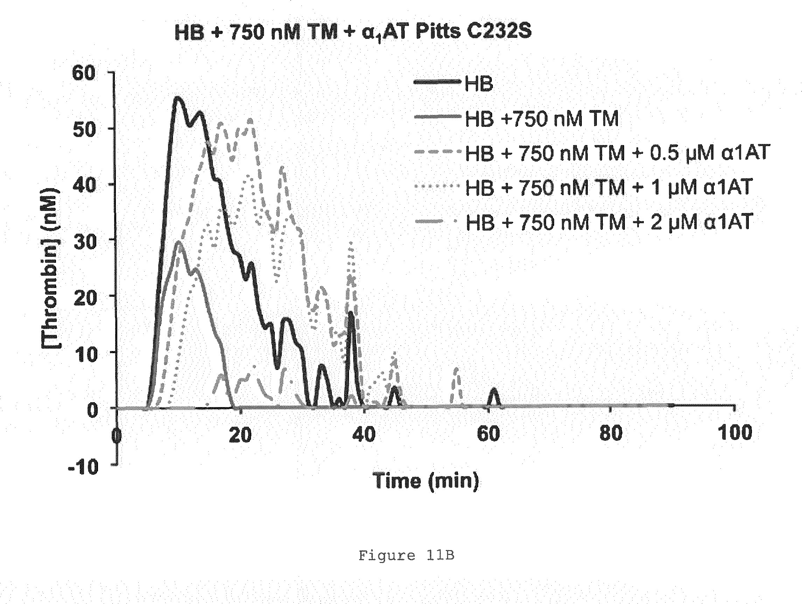

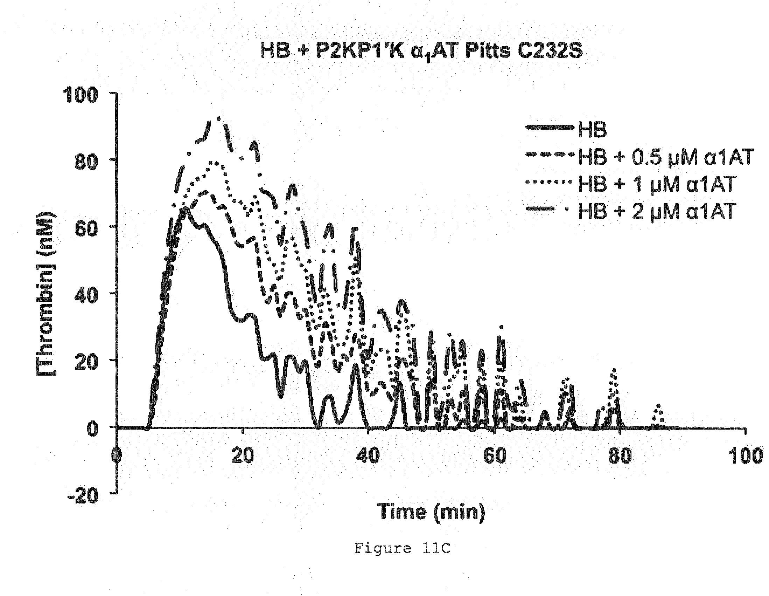

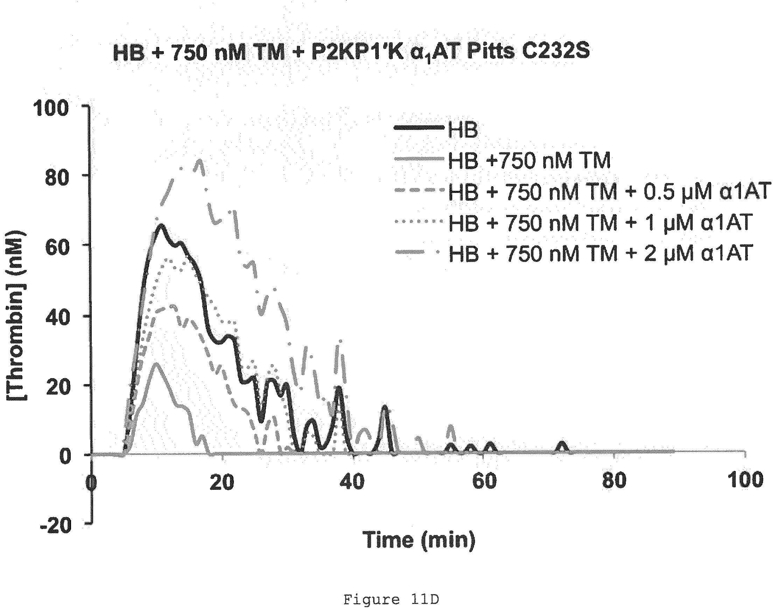

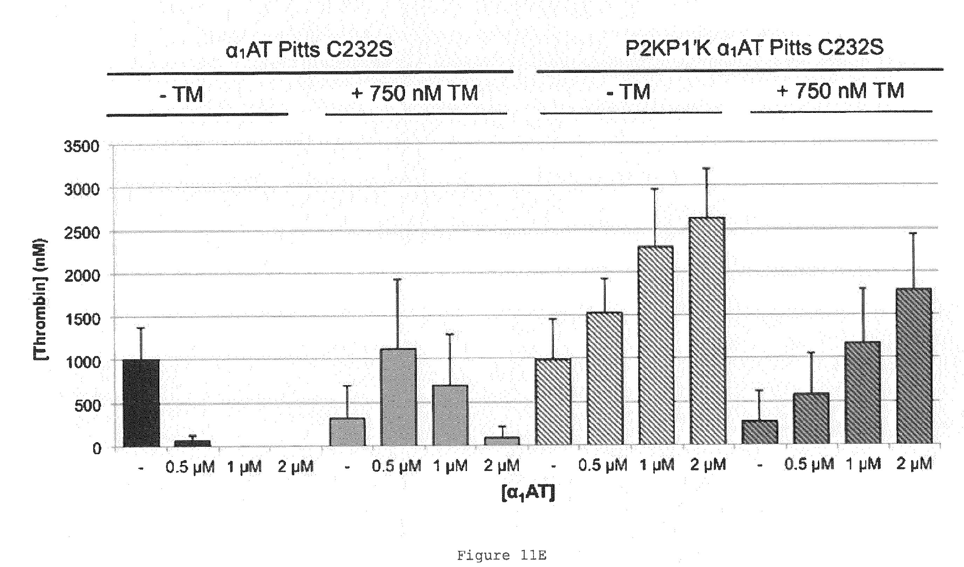

Figure 11 shows the effect of FL α₁AT Pitts C232S and FL α₁AT Pitts C232S P2KP1'K in HB mouse plasma. Fig 11A-D show representative thrombin generation curves for reactions containing increasing concentrations of (A-B) FL α₁AT Pitts C232S or (C-D) FL α₁AT Pitts C232S P2KP1'K in the presence of (A and C) no TM (B and D) 750 nM soluble human TM. All assays were performed in HB mouse plasma collected by tail bleed into citrate, centrifuged to remove red cells and frozen at -80°C. Coagulation was initiated by the addition of CaCl₂, TF/phospholipid (RB low TF and phospholipid reagent, Technoclone) and 1:12,000 Dade Innovin (Siemens) to activate coagulation through the extrinsic pathway. Thrombin generation was measured through the cleavage of a fluorogenic substrate (Z-Gly-Gly-Arg-AMC). Fluorescence units were converted to thrombin concentration by calibrating fluorescence units against known concentrations of thrombin, using the Technothrombin calibration kit (Technoclone). Fig 11E shows mean ETPs (endogenous thrombin potentials), representing the total amount of thrombin generated during the reactions. Bars show the mean of four independent experiments. Error bars represent the standard deviation. All assays were performed at 33°C.

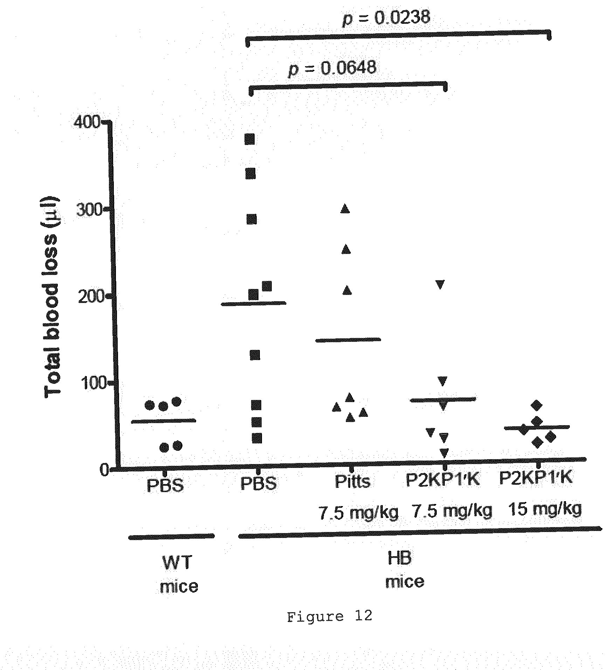

Figure 12 shows that FL α₁AT Pitts C232S P2KP1'K reduces bleeding in HB mice in a tail clip assay. Tail clip results show the total blood loss over a 10 min collection period after tail transection at 3 mm diameter for WT mice or HB mice. Mice were injected either with PBS, FL α₁AT Pitts C232S or FL α₁AT Pitts C232S P2KP1'K at the indicated doses in a total injection volume of 200 µl. Protein solutions were made up to 200 µl using PBS. The volume blood loss was determined by collection of blood from the tail into 14 ml saline. The collected blood was spun down and the red cells lysed, followed by measurement of the absorbance at 575 nm. A standard curve was constructed by determining the absorbance at 575 nm after red cell lysis using known volumes of collected blood. Blood loss in the experimental samples was then calculated from this standard. Each point shown indicates data from a single mouse and the horizontal lines show the mean of all animals per group. P values were calculated using an unpaired t-test. Circles indicate WT mice injected with PBS, squares indicate HB mice injected with PBS, triangles indicate HB mice injected with 7.5 mg/kg FL α₁AT Pitts C232S, inverted triangles indicate HB mice injected with 7.5 mg/kg FL α₁AT Pitts C232S P2KP1'K and diamonds indicate HB mice injected with 15 mg/kg FL α₁AT Pitts C232S P2KP1'K.

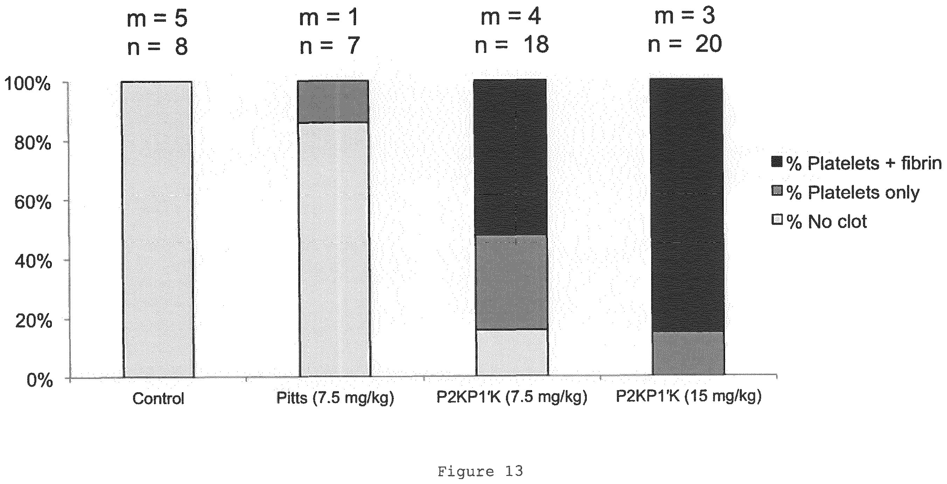

Figure 13 shows that FL α₁AT Pitts C232S P2KP1'K increases stable clot formation in HB mice in a cremaster arteriole laser injury model. HB mice were infused with a fluorescently tagged α-fibrin antibody and a fluorescently tagged antibody for labeling platelets through a jugular vein cannula. Controls indicate the baseline level of clot formation after injury in mice infused with antibodies only. m indicates the number of mice per condition, n indicates the number of injuries performed for that condition. Light grey indicates no clot, dark grey indicates a platelet only clot, and black represents clots containing platelets and fibrin.

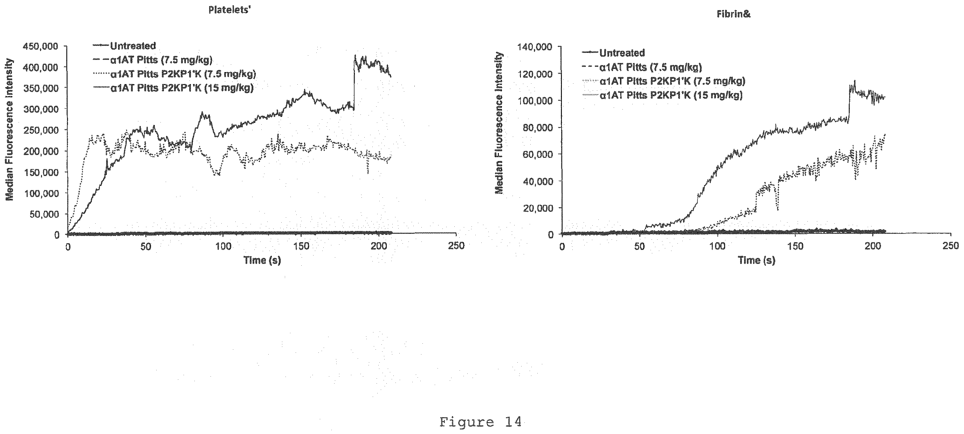

Figure 14 shows that FL α₁AT Pitts C232S P2KP1'K increases stable clot formation in HB mice in a cremaster arteriole laser injury model. Figure 14 shows representative raw data of the results shown in Figure 13. Fluorescence was quantified over time from all injuries for each condition. Graphs display the median value from all injuries in the indicated condition. Number of mice and total number of injuries were as follows: Control: 5 mice, 8 injuries; FL α₁AT Pitts C232S at 7.5 mg/kg: 1 mouse, 7 injuries; FL α₁AT Pitts C232S P2KP1'K at 7.5 mg/kg: 4 mice, 18 injuries; FL α₁AT Pitts C232S P2KP1'K at 15 mg/kg: 3 mice, 20 injuries.

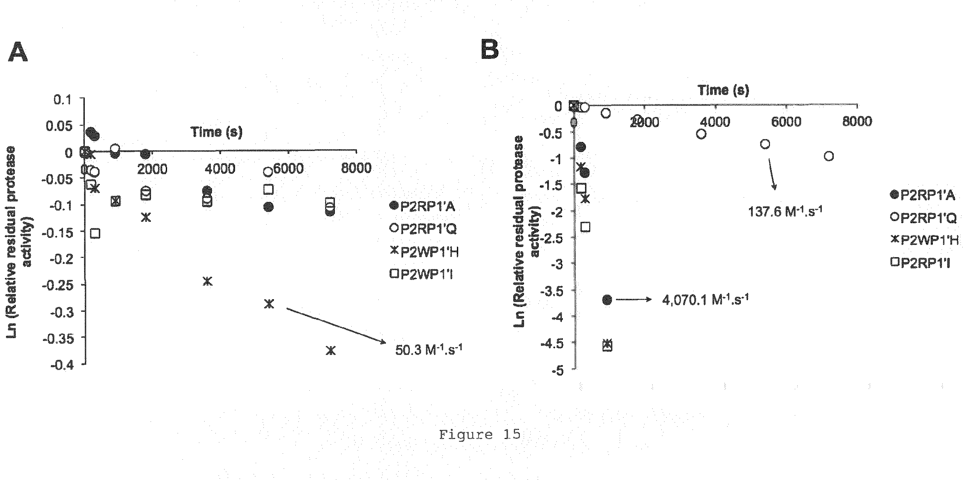

Figure 15 shows the inhibition of thrombin and fXa by mutants of α₁AT specific for APC over thrombin that were rescreened for fXa inhibition. The resulting four mutants, which should have reduced fXa inhibition were then tested for inhibition of (A) thrombin and (B) fXa, to determine if the screening had been successful in producing APC-specific mutants which did not inhibit thrombin or fXa. 1 µM serpin was incubated with 12.5 nM protease for different amounts of time. At the indicated timepoints, the reaction was stopped by addition of an excess of chromogenic substrate (S2238 for thrombin, S2222 for fXa). The residual enzyme activity was divided by the initial enzyme activity and the natural log of this value plotted against time. The slope of these plots is the observed rate constant, k_{obs}. To obtain an estimate of the second-order rate constant k₂, k_{obs} was divided by the serpin concentration. Selected values are shown in the figure, to illustrate the highest inhibition of thrombin and fXa, as well as the value for the only mutant that did not substantially inhibit fXa (P2RP1'Q). The mutants shown all have the Pitts (M358R, P1R) mutation.

### Detailed Description

This disclosure relates to the modification of serpins to increase their specificity for APC relative to other coagulation proteases. Modified serpin variants specific for APC may be useful, for example as procoagulants.

Since they are specific to APC, the modified serpins described herein are expected to have few or no off-target effects and are specific to pathways that are activated on injury. This allows the modified serpins to be applied therapeutically and prophylactically, as well as in emergency situations, i.e. trauma. The dosing of modified serpins is predictable since they are suicide inhibitors, meaning that one molecule of serpin cannot inhibit more than one molecule of protease. In addition, because efficient natural clearance mechanisms are specific for serpin: protease complexes over serpins alone, the plasma half-lives of modified serpins are likely to exceed those of current bypassing agents. For example, the half-life of a modified serpin as described herein may be about 5 days.

Protein C (Gene ID 5624) is a vitamin K-dependent plasma glycoprotein that is cleaved to its activated form (activated Protein C, APC) by the thrombin-thrombomodulin complex. Human Protein C has the reference amino acid sequence of NP_000303.1 GI: 4506115 and may be encoded by the reference nucleotide sequence of NM_000312.3 GI: 270483720. APC is an anticoagulant protease that proteolytically cleaves fVa and fVIIIa (Figure 1), thereby attenuating the production of thrombin.

A modified serpin as described herein may have mutations in its reactive center loop (RCL); the residues at both of positions P1' and P2 and optionally P1 and/or P4 may be mutated.

RCL residues are numbered herein according to the Schechter-Berger nomenclature for substrates and inhibitors of serine proteases (Schechter & Berger, 1967). This standard nomenclature allows the residue at specific positions in the RCL, such as positions P1', P1, P2 and/or P4, to be easily identified in any serpin sequence.

Preferably, these mutations are the only mutations in the RCL of the modified serpin. For example, the RCL may consist of the sequence of the RCL of the wild-type serpin with mutations at positions P1' and P2 and optionally P1 and/or P4.

The RCL of the modified serpin may have mutations at the P1' and P2 positions; mutations at the P1', P2 and P1 positions; mutations at the P1', P2 and P4 positions or_mutations at the P1', P2, P1 and P4 positions. The residues at other positions in the RCL may be unmutated wild-type residues.

Preferably, the residues at positions P1', P1 and P2 are mutated.

The reactive center loop (RCL) of a serpin is typically about 20 residues in length and contains the scissile P1-P1' bond that is cleaved by the target protease. The RCL extends from strand 5 of beta sheet A to strand 1 of beta sheet C of the serpin. Residues P17 Glu, P15 Gly and P14 Thr are conserved in serpins. For example, the RCL of a serpin may comprise the consensus sequence P17 E, P16 E/K/R, P15 G, P14 T/S, P12-P9 (A/G/S)₄ (Hopkins et al. 1993; Irving et al. 2000). The RCL starts at residue P17 and usually ends at residue P3'. RCLs may be extended in some serpins, such as PCI, by additional residues on the P' side. For example, the RCL of α₁-antitrypsin consists of residues P17-P3' and the RCL of PCI consists of residues P17-P6'. Examples of serpins with residues P1', P1, P2 and P4 highlighted are shown in SEQ ID NOS: 1 to 11 below. The residues that constitute the mature serpin sequences are also indicated.

The residues in the other positions of the RCL in the serpin may be unmodified i.e. they may be the native residues of the wild-type serpin sequence. The modified serpin may therefore comprise an RCL having a wild-type sequence with mutations at positions P1, P1', P2 and/or P4 as described above.

The mutations in the reactive center loop (RCL) of the modified serpin may comprise a mutation at the P1' position. Preferably, the mutation is a substitution. The native P1' residue in the RCL of the wild-type serpin may be replaced with a non-native residue in the modified serpin. For example, the native S residue at the P1' position in the wild-type sequence of α₁AT or PCI may be replaced with a residue other than S in the modified serpin.

The native P1' residue in the RCL of the wild-type serpin may be replaced with K, R, or Q; most preferably K.

The mutations in the reactive center loop (RCL) of the modified serpin may comprise a mutation at the P2 residue. Preferably, the mutation is a substitution. For example, the native P2 residue in the RCL of the wild-type serpin may be replaced with a non-native residue in the modified serpin.

The native P2 residue in the RCL of the wild-type serpin may be replaced with R, H or K, preferably K.

The mutations in the RCL of the modified serpin may comprise or consist of substitutions at the P1' and P2 residues i.e. the residues located at the P1' and P2 positions in the RCL of the wild-type serpin sequence may be replaced by other residues in the modified serpin.

Suitable residues at the P2 and P1' positions of the modified serpin are described above.

Examples of P2 and the P1' residues respectively in a modified serpin as described herein include KK, RK, RH, KH, RQ or KQ and TQ.

In some preferred embodiments, both the P1' and the P2 residue may be modified to positively charged residues, such as K, H or R, most preferably K.

In some embodiments, the P1 residue may be unmodified in the modified serpin. For example, the residue at the P1 position in the RCL of the modified serpin may be the residue that is present at the P1 residue in the wild-type serpin sequence. For example, the P1 residue in a modified PCI may be an R residue.

In other embodiments, the P1 residue may be mutated in the modified serpin. For example, the one or more mutations in the reactive center loop (RCL) of the modified serpin further comprise a mutation at the P1 residue. Preferably, the mutation is a substitution. The native P1 residue in the RCL of the wild-type serpin may be replaced with a non-native residue in the modified serpin.

In some embodiments, the P1 residue may be mutated or modified to a positively charged residue such as H, K or R, preferably R.

Preferably, a native residue that is non-positively charged at the P1 position of a wild-type serpin may be replaced by a positively charged residue in the modified serpin. For example, M at the P1 position of wild-type α₁AT may be replaced by a positively charged residue, such as R, in a modified α₁AT. The Pittsburgh (Pitts) variant of α₁AT has a mutation at residue 358 which replaces the M residue at the P1 position with an R residue.

In some embodiments, the P4 residue may be unmodified in the modified serpin. For example, the residue at the P4 position in the RCL of the modified serpin may be the residue that is present at the P4 residue in the wild-type serpin sequence. For example, the P4 residue in a PCI scaffold may be F and the P4 residue in an α₁AT scaffold may be A.

In other embodiments, the P4 residue may be mutated in the modified serpin. For example, the one or more mutations in the reactive center loop (RCL) of the modified serpin further comprise a mutation at the P4 residue.

Preferably, the mutation is a substitution. The residue at the P4 residue in the RCL of the wild-type serpin may be replaced with a different residue in the modified serpin. For example, the P4 residue in a modified PCI may be mutated or modified to a residue other F and the P4 residue in a modified α₁AT may be mutated or modified to a residue other than A.

Suitable residues in the P4 position of the RCL of the modified serpin include S, R, V, C, W, K, G, L, H, F, T, Q and A.

In examples of modified procoagulant serpins as described herein,
(1) the P4 residue is K, the P2 residue is R, the P1 residue is R and the P1' residue is H;
(2) the P4 residue is F, the P2 residue is K, the P1 residue is R and the P1' residue is K;
(3) the P4 residue is F, the P2 residue is R, the P1 residue is R and the P1' residue is K;
(4) the P4 residue is S, the P2 residue is H, the P1 residue is R and the P1' residue is R;
(5) the P4 residue is A, the P2 residue is R, the P1 residue is R, and the P1' residue is H;
(6) the P4 residue is A, the P2 residue is K, the P1 residue is R, and the P1' residue is H;
(7) the P4 residue is A, the P2 residue is K, the P1 residue is R, and the P1' residue is K;
(8) the P4 residue is A, the P2 residue is K, the P1 residue is R, and the P1' residue is Q, or
(9) the P4 residue is A, the P2 residue is R, the P1 residue is R, and the P1' residue is Q.

In some preferred modified serpins, the P4 residue is A, the P2 residue is K, the P1 residue is R, and the P1' residue is K.

In some disclosure herein, the combination of residues at the P4, P2 and the P1' positions in a modified procoagulant serpin as described herein may be non-naturally occurring i.e. the combination of residues at the P4, P2 and the P1' positions is not found in the parent wild-type (i.e. unmodified) serpin or in other wild-type serpins.

A modified serpin as described herein may comprise the sequence of a wild-type (i.e. unmodified) serpin, preferably a mature wild-type serpin, with one or more mutations in the RCL thereof as described above, and optionally one or more additional mutations outside the RCL.

The sequences of wild-type serpins are well-known in the art, and may include SEQ ID NOS: 1 to 11 as set out herein. The sequences of wild-type serpins may include the sequence of mature wild-type proteins.

The mature protein C inhibitor (PCI) sequence including its propeptide corresponds to residues 20 to 406 of SEQ ID NO: 1. The mature α₁-antichymotrypsin corresponds to residues 26 to 423 of SEQ ID NO: 2. The mature C1-esterase inhibitor sequence corresponds to residues 23-500 of SEQ ID NO: 3. The mature α₂-antiplasmin sequence corresponds to residues 28-491 of SEQ ID NO: 4. The mature antithrombin (ATIII) sequence corresponds to residues 33-464 of SEQ ID NO: 5. The mature heparin cofactor II sequence corresponds to residues 20-499 of SEQ ID NO: 6. The mature α₁-antitrypsin (α₁AT) sequence corresponds to residues 25-418 of SEQ ID NO: 7. The mature kallistatin sequence corresponds to residues 21-427 of SEQ ID NO: 8. The mature plasminogen activator inhibitor sequence corresponds to residues 24-402 of SEQ ID NO: 9. The mature protein Z dependent inhibitor sequence corresponds to residues 22-444 of SEQ ID NO: 10. The mature protease nexin 1 isoform a sequence corresponds to residues 20-398 of SEQ ID NO: 11.

Other than mutations of residues in the RCL as described above, a modified serpin may have 50 or fewer amino acid residues altered relative to a wild-type serpin amino acid sequence (for example the mature serpin sequence of one of SEQ ID NOS 1 to 11, preferably SEQ ID NO: 1 or 7), preferably 45 or fewer, 40 or fewer, 30 or fewer, 20 or fewer, 15 or fewer, 10 or fewer, 5 or fewer or 3 or fewer. For example, a modified serpin may comprise the sequence of a wild-type serpin with 50 or fewer, 45 or fewer, 40 or fewer, 30 or fewer, 20 or fewer, 15 or fewer, 10 or fewer, 5 or fewer or 3 or fewer amino acid residues mutated or altered, in addition to the one, two, three or four amino acid residues in the RCL of the serpin that are mutated or altered as described above (i.e. the residues at positions P1' and/or P2 and optionally P1 and/or P4).

An amino acid residue in the wild-type amino acid sequence may be altered or mutated by insertion, deletion or substitution, preferably substitution for a different amino acid residue. Such alterations may be caused by one or more of addition, insertion, deletion or substitution of one or more nucleotides in the encoding nucleic acid.

For example, a modified serpin may comprise the amino acid sequence of residues 25-418 of SEQ ID NO: 12 having 50 or fewer mutations, wherein said mutations are at positions other than P4, P2, P1 and P1' i.e. the P4 residue at in the RCL of the modified serpin is A, the P2 residue is K, P1 residue is R and the P1' residue is K.

The P4 residue in the modified serpin of SEQ ID NO: 12 is located at position 379 (355 of the mature protein), the P2 residue is located at position 381 (357 of the mature protein), the P1 residue is located at position 382 (358 of the mature protein), and the P1' residue is located at position 383 (359 of the mature protein).

The modified serpin may share at least 50% sequence identity with the wild-type amino acid sequence of a wild-type serpin, for example the mature serpin sequence of any one of SEQ ID NOS: 1 to 11, preferably SEQ ID NO: 1 or 7, at least 55%, at least 60%, at least 65%, at least 70%, at least about 80%, at least 90%, at least 95%, at least 98% or at least 99% sequence identity.

For example, a modified serpin may comprise an amino acid sequence having at least 50% sequence identity to residues 25-418 of SEQ ID NO: 12, wherein the P4 residue in the RCL of the modified serpin is A, the P2 residue is K, P1 residue is R and the P1' residue is K.

Sequence identity is commonly defined with reference to the algorithm GAP (Wisconsin GCG package, Accelerys Inc, San Diego USA). GAP uses the Needleman and Wunsch algorithm to align two complete sequences that maximizes the number of matches and minimizes the number of gaps. Generally, default parameters are used, with a gap creation penalty = 12 and gap extension penalty = 4. Use of GAP may be preferred but other algorithms may be used, e.g. BLAST (which uses the method of Altschul et al. (1990) J. Mol. Biol. 215: 405-410), FASTA (which uses the method of Pearson and Lipman (1988) PNAS USA 85: 2444-2448), or the Smith-Waterman algorithm (Smith and Waterman (1981) J. Mol Biol. 147: 195-197), or the TBLASTN program, of Altschul et al. (1990) supra, generally employing default parameters. In particular, the psi-Blast algorithm may be used (Nucl. Acids Res. (1997) 25 3389-3402). Sequence identity and similarity may also be determined using Genomequest™ software (Gene-IT, Worcester MA USA).

Sequence comparisons are preferably made over the full-length of the relevant sequence described herein.

Preferably, a modified procoagulant serpin as described herein comprises the RCL consensus P17 E, P16 S/E/K/R, P15 G, P14 T/S, P12-P9 (A/G/S)₄.

A modified serpin may further comprise one or more residues that are conserved in wild-type serpin sequences. For example, a modified serpin may comprise some or all of the following residues (numbered according to their position in α1AT): 33F, 49N, 53S, 54P, 56S, 61L, 67G, 72T, 80L, 130F, 147F, 157I, 158N, 161V, 165T, 167G, 169I, 180T, 184L, 186N, 190F, 191K, 192G, 194W, 198F, 203T, 208F, 218V, 220M, 221M, 277Y, 254L, 255P, 289P, 290K, 299L, 303L, 307G, 312F, 316A, 327L, 334H, 342E, 344G, 347A, 369P, 370F, 383L, 384F, 386G, and 391P (Irving et al 2008). The corresponding conserved residues in other serpin sequences may be readily determined using routine sequence analysis.

Mutations or variations outside the RCL of the modified serpin may include replacement of one or more Cys residues in the modified serpin, such as the C232 (numbering according to the mature sequence) residue of α₁AT, to abolish disulfide bridge formation or other modifications; deletion or substitution of residues at the N terminus of the wild-type sequence, for example to facilitate expression; or mutation or modification of residues in the heparin binding sites of the modified serpins (i.e. helix D or helix H) in order to alter the heparin binding activity of the modified serpin.

In some disclosure herein, the modified serpin may have an N terminal truncation relative to the wild-type serpin. For example, the modified serpin may have a 10 to 30 residue truncation at the N terminus, preferably about 20 residues.

One or more residues in the modified serpin may be non-natural amino acids, modified amino acids or D-amino acids. The use of such amino acids is well-known to those of skill in the art

A modified serpin as described herein may display the secondary structure of the wild-type serpin, for example a modified serpin may display a structure comprising 3 beta sheets, 8-9 alpha helices and a flexible RCL of about 20 residues.

In some preferred disclosure herein, the modified serpin may consist of the amino acid sequence of a wild-type serpin with one or more mutations in the reactive center loop (RCL) thereof as described herein.

Preferably, the modified serpin is non-immunogenic in a human. For example, the wild-type serpin may be a human serpin, preferably a human plasma serpin.

The wild-type serpin may be α₁-antichymotrypsin (SERPINA3), α₂-antiplasmin (SERPINF2) antithrombin (ATIII) (SERPINC1), heparin cofactor II (HCII) (SERPIND1), protein C inhibitor (PCI) (SERPINA5), α₁-antitrypsin (α₁AT) (SERPINA1), Kallistatin (SERPINA4), Plasminogen activator inhibitor-1 (SERPINE1), C1-esterase inhibitor (SERPING1), protease nexin 1 (SERPINE2) or Protein Z-dependent inhibitor (SERPINA10) (Whisstock et al JBC. 285 32 24307-24312, Rau et al Journal of Thrombosis and Hemostasis, 5 (Suppl. 1): 102-115, Huntington, Journal of Thrombosis and Hemostasis, 9 (Suppl. 1): 26-34) .

Preferably, the wild-type serpin is ATIII, HCII, PCI or α₁AT, most preferably PCI or α₁AT (Huntington et al Cell. Mol. Life Sci. 66 (2009) 113-121; Li et al JBC. 283 51 36039-36045; and Li et al PNAS 2008 105 4661-4666).

α₁-antichymotrypsin (SERPINA3; Gene ID 12) may have the reference amino acid sequence of NP_001076.2 GI:50659080 (SEQ ID NO: 2) and may be encoded by the reference nucleotide sequence of NM_001085.4 GI:73858562.

C1-esterase inhibitor (SERPING1; Gene ID 710) may have the reference amino acid sequence of NP_000053.2 GI: 73858568(SEQ ID NO: 3) and may be encoded by the reference nucleotide sequence of NM_000062.2 GI:73858567.

α₂-antiplasmin (SERPINF2 Gene ID 5345) may have the reference amino acid sequence of NP_000925.2 GI:115583663(SEQ ID NO: 4)and may be encoded by the reference nucleotide sequence of NM_001165920.1 GI:260064047

Antithrombin (ATIII) (SERPINC1 Gene ID 462) may have the reference amino acid sequence of NP_000479.1 GI: 4502261 (SEQ ID NO: 5) and may be encoded by the reference nucleotide sequence of NM_000488.3 GI:254588059

Heparin cofactor II (HCII) (SERPIND1 Gene ID3053) may have the reference amino acid sequence of NP_000176.2 GI: 73858566 (SEQ ID NO: 6) and may be encoded by the reference nucleotide sequence of NM_000185.3 GI:73858565

Protein C inhibitor (PCI) (SERPINA5 Gene ID 5104) may have the reference amino acid sequence of NP_000615.3 GI: 194018472 and may be encoded by the reference nucleotide sequence of NM_000624.5 GI:401782581. In some disclosure herein, the protein C inhibitor (PCI) may be an allelic variant resulting from a substitution at SNP rs6115, VAR_013081 (the S45N variant, numbering according to mature protein including the propeptide) and having the sequence shown in SEQ ID NO: 1. Residues 1-19 of SEQ ID NO: 1 correspond to the signal sequence. In plasma, PCI may exist in a full-length form which includes the propeptide of residues 20-25 of SEQ ID NO: 1 (i.e. residues 20-406 of SEQ ID NO: 1) or N terminally cleaved form which lacks the propeptide (i.e. residues 26-406 of SEQ ID NO: 1).

α₁-antitrypsin (α₁AT) (SERPINA1 Gene ID 5265) may have the reference amino acid sequence of NP_000286.3 GI:50363217(SEQ ID NO: 7) and may be encoded by the reference nucleotide sequence of NM_000295.4 GI:189163524.

Kallistatin (SERPINA4 Gene ID 5267) may have the reference amino acid sequence of NP_006206.2 GI: 21361302(SEQ ID NO: 8) and may be encoded by the reference nucleotide sequence of NM_006215.2 GI: 21361301.

Plasminogen activator inhibitor-1 (SERPINE1 Gene ID 5054) may have the reference amino acid sequence of NP_000593.1 GI: 10835159(SEQ ID NO: 9) and may be encoded by the reference nucleotide sequence of NM_000602.4 GI: 383286745.

Protein Z-dependent inhibitor (PZI) (SerpinA10; Gene ID 51156) may have the reference amino acid sequence of NP_057270.1 GI: 7705879 (SEQ ID NO: 10) and may be encoded by the reference nucleotide sequence of NM_016186.2 GI: 154759289.

Protease nexin 1 (PN1) (SerpinE2; Gene ID 5270) may have the reference amino acid sequence of NP_001130000.1 GI: 24307907, NP_001130002.1 GI: 211904152 or NP_006207.1 GI: 211904156 (SEQ ID NO: 11) and may be encoded by the reference nucleotide sequence of NM_001136528.1 GI: 211904151, NM_001136530.1 GI: 211904155 or NM_006216.3 GI: 211904150.

The P1', P1, P2 and P4 residues that may be mutated as described above are highlighted in bold in SEQ ID NOS: 1 to 11.

The one or more mutations in the RCL alter the specificity of the modified serpin relative to the unmodified wild-type serpin. The modified serpin displays increased selectively for anticoagulant proteases over procoagulant proteases compared to the wild-type serpin.

Preferably, the one or more mutations within the RCL increase the inhibition of APC by the modified serpin relative to the inhibition of other coagulation proteases, in particular one or more procoagulant proteases out of thrombin, fXa, fVIIa, fIXa and fXIa.

For example, the one or more mutations in the RCL of the modified serpin may increase the inhibition of APC by the modified serpin relative to the inhibition of thrombin. The selective inhibition of APC relative to thrombin may be increased in the presence or absence of heparin.

In addition, the one or more mutations in the RCL of the modified serpin may increase the inhibition of APC by the modified serpin relative to the inhibition of 1, 2, 3 or all 4 of the procoagulant proteases fXa, fVIIa, fIXa and fXIa.

A serpin modified as described herein displays greater inhibition of APC relative to thrombin and other procoagulant proteases than the unmodified wild-type serpin.

The modified serpin may show greater inhibition of APC than inhibition of thrombin. For example, inhibition of APC by the modified serpin may be at least 5 fold more, at least 10 fold more at least 100 or at least 1000 fold more than inhibition of thrombin by the modified serpin. In some embodiments, the modified serpin may inhibit APC with a second-order rate constant (k₂) that is at least 5 fold more, at least 10 fold more at least 100 or at least 1000 fold more than the second-order rate constant for the inhibition of thrombin. Preferably the stoichiometry of inhibition of the modified serpin for APC is 1.

Preferably, a modified serpin as described herein may bind and inhibit APC but display no binding or inhibition or substantially no binding or inhibition of thrombin.

The one or more mutations in the RCL may also increase the inhibition of APC relative to the inhibition of 1, 2, 3, or all 4 of fVIIa, fIXa, fXa and fXIa. Inhibition of APC relative to fVIIa, fIXa, fXa and/or fXIa may be increased in the presence or absence of heparin.

For example, the modified serpin may display greater inhibition of APC relative to 1, 2, 3, or all 4 of fVIIa, fIXa, fXa and fXIa than the wild-type serpin.

The modified serpin may inhibit APC more than it inhibits fVIIa. For example, inhibition of APC by the modified serpin may be at least 2 fold more, at least 10 fold more, at least 100 more, or at least 1000 fold more than the inhibition of fVIIa by the modified serpin. The modified serpin inhibits APC with a second-order rate constant (k₂) that is at least 2 fold more, at least 10 fold more, at least 100 more, or at least 1000 fold more than the second-order rate constant for the inhibition of fVIIa.

The modified serpin may inhibit APC more than it inhibits fIXa. For example, inhibition of APC by the modified serpin may be at least 2 fold more, at least 10 fold more, at least 100 more or at least 1000 fold more than the inhibition of fIXa by the modified serpin. The modified serpin inhibits APC with a second-order rate constant (k₂) that is at least 2 fold more, at least 10 fold more, at least 100 more, or at least 1000 fold more than the second-order rate constant for the inhibition of fIXa.

The modified serpin may inhibit APC more than it inhibits fXa. For example, inhibition of APC by the modified serpin may be at least 1.5 fold more, at least 2 fold more, at least 10 fold more at least 100 or at least 1000 fold more than the inhibition of fXa by the modified serpin. The modified serpin inhibits APC with a second-order rate constant (k₂) that is at least 1.5 fold more, at least 2 fold more, at least 10 fold more, at least 100 more, or at least 1000 fold more than the second-order rate constant for the inhibition of fXa.

The modified serpin may inhibit APC more than it inhibits fXIa. For example, inhibition of APC by the modified serpin may be at least 2 fold more, at least 10 fold more at least 100 or at least 1000 fold more than the inhibition of fXIa by the modified serpin. The modified serpin inhibits APC with a second-order rate constant (k₂) that is at least 2 fold more, at least 10 fold more, at least 100 more, or at least 1000 fold more than the second-order rate constant for the inhibition of fXIa.

A modified serpin as described herein may be part of a fusion protein which contains one or more heterologous amino acid sequences additional to the modified serpin sequence. For example, the fusion protein comprising the modified serpin may further comprise one or more additional domains which improve the stability, pharmacokinetic, targeting, affinity, purification and production properties of the modified serpin.

Suitable additional domains include immunoglobulin Fc domains. Immunoglobulin Fc domains are well-known in the art and include the human IgG1 Fc domain. A human immunoglobulin Fc domain may be located at the N-terminal or C- terminal end of the modified serpin.

Modified serpins as described herein may be provided using synthetic or recombinant techniques which are standard in the art.

In some disclosure herein, the modified serpin may be produced as a fusion protein further comprising an affinity tag, which may, for example, be useful for purification. An affinity tag is a heterologous peptide sequence which forms one member of a specific binding pair. Polypeptides containing the tag may be purified by the binding of the other member of the specific binding pair to the polypeptide, for example in an affinity column. For example, the tag sequence may form an epitope which is bound by an antibody molecule.

Suitable affinity tags include for example, glutathione-S-transferase, (GST), maltose binding domain (MBD), MRGS(H)₆, DYKDDDDK (FLAG™), T7-, S-(KETAAAKFERQHMDS), poly-Arg(R₅₋₆), poly-His(H₂₋₁₀), poly-Cys(C₄)poly-Phe(F₁₁)poly-Asp(D₅₋₁₆), SUMO tag (Invitrogen Champion pET SUMO expression system), Strept-tag II (WSHPQFEK), c-myc (EQKLISEEDL), Influenza-HA tag (Murray, P. J. et al (1995) Anal Biochem 229, 170-9), Glu-Glu-Phe tag (Stammers, D. K. et al (1991) FEBS Lett 283, 298-302), Tag.100 (Qiagen; 12 aa tag derived from mammalian MAP kinase 2), Cruz tag 09™ (MKAEFRRQESDR, Santa Cruz Biotechnology Inc.) and Cruz tag 22™ (MRDALDRLDRLA, Santa Cruz Biotechnology Inc.). Known tag sequences are reviewed in Terpe (2003) Appl. Microbiol. Biotechnol. 60 523-533. In preferred disclosure herein, a poly-His tag such as (H)₆, His-SUMO tag (Invitrogen Champion pET SUMO expression system), or MRGS(H)₆ may be used.

The affinity tag sequence may be separated from the modified serpin after purification, for example, using site-specific proteases.

In some disclosure herein, the modified serpin may be coupled to an appropriate signal leader peptide to direct secretion of the fusion polypeptide from cell into the culture medium. A range of suitable signal leader peptides are known in the art. The signal leader peptide may be a serpin signal sequence or may be heterologous to the modified serpin i.e. it may be a non-serpin signal sequence. For example, an α-factor secretion signal or BiP signal sequence may be employed. Preferably, the signal peptide is removed by post-translational processing after expression of the polypeptide.

Modified serpins as described herein may be isolated, in the sense of being free from contaminants, such as unmodified serpins and other polypeptides and/or serum components.

Modified serpins as described herein may inhibit one or more activities of activated protein C (APC). For example, modified serpins as described herein may inhibit the proteolytic cleavage of one or more APC substrates, such as fVa or fVIIIa. For example, binding of the modified serpin to APC may result in an at least 5-fold, at least 10-fold, or at least 15-fold decrease in the proteolytic cleavage of fVa, fVIIIa and/or another APC substrate. In some embodiments, binding of APC by the modified serpin may result in no detectable cleavage of fVa or fVIIIa substrate by APC.

Techniques for measuring APC activity, for example by measuring the proteolytic cleavage of APC substrates *in vitro* are standard in the art and are described herein. Suitable assays for use in determining APC activity include standard kinetic assays, for example to measure rate constants, and coagulation assays, including thrombin generation assays (TGA).

Techniques for measuring the activity of procoagulant proteases, for example by measuring the proteolytic cleavage of chromogenic substrates *in vitro* are standard in the art and are described herein. Suitable assays for use in determining protease activity include standard kinetic assays, for example to measure rate constants, and coagulation assays, including thrombin generation assays (TGA), prothrombin time assays (PT) and activated partial thromboplastin time assays (aPTT).

In some disclosure herein, modified serpins as described herein may be further modified by chemical modification, for example by PEGylation, or by incorporation in a liposome, to improve their pharmaceutical properties, for example by increasing *in vivo* half-life.

A modified serpin as described herein may be attached to one or more polyethylene glycol (PEG) or other moieties to increase the in vivo half-life of the modified serpin (Cantin et al. 2002, Am. J. Respir. Cell Mol. Biol. 27; 659-665). For example, a modified serpin may be mono-pegylated or poly-pegylated (for example, with 2-6 PEG moieties). Suitable pegylation methods are well known in the art.

The effect of a modified serpin on coagulation and bleeding may be determined. Suitable techniques are standard in the art. For example, the effect of a modified serpin on thrombin generation may be determined using a thrombin generation assay (TGA) or an activated partial thromboplastin time assay or a prothrombin time assay described herein. Suitable *in vivo* models include cremaster arteriole laser injury models, FeCl₃ carotid artery models and tail clip assays as described herein. Other suitable coagulation models are well known in the art.

Other aspects of the invention provide a nucleic acid encoding a modified serpin as described above and a vector comprising such a nucleic acid.

Suitable vectors can be chosen or constructed, containing appropriate regulatory sequences, including promoter sequences, terminator fragments, polyadenylation sequences, enhancer sequences, marker genes and other sequences as appropriate. Preferably, the vector contains appropriate regulatory sequences to drive the expression of the nucleic acid in mammalian cells. A vector may also comprise sequences, such as origins of replication, promoter regions and selectable markers, which allow for its selection, expression and replication in bacterial hosts such as *E. coli.*

Vectors may be plasmids, viral e.g. phage, or phagemid, as appropriate. For further details see, for example, Molecular Cloning: a Laboratory Manual: 3rd edition, Russell et al., 2001, Cold Spring Harbor Laboratory Press. Many known techniques and protocols for manipulation of nucleic acid, for example in preparation of nucleic acid constructs, mutagenesis, sequencing, introduction of DNA into cells and gene expression, are described in detail in Current Protocols in Molecular Biology, Ausubel et al. eds. John Wiley & Sons, 1992.

A nucleic acid or vector as described herein may be introduced into a host cell.

Another aspect of the invention provides a recombinant cell comprising a nucleic acid or vector that expresses a polypeptide comprising or consisting of a modified serpin as described above.

A range of host cells suitable for the production of recombinant modified serpins are known in the art. Suitable host cells may include prokaryotic cells, in particular bacteria such as *Escherichia coli* and *Lactococcus lactis* and eukaryotic cells, including mammalian cells such as CHO and CHO-derived cell lines (Lec cells), HeLa, COS, HEK293 and HEK-EBNA cells, amphibian cells such as Xenopus oocytes, insect cells such as *Trichoplusia ni,* Sf9 and Sf21 and yeast cells, such as *Pichia pastoris.*

Techniques for the introduction of nucleic acid into cells are well established in the art and any suitable technique may be employed, in accordance with the particular circumstances. For eukaryotic cells, suitable techniques may include calcium phosphate transfection, DEAE-Dextran, electroporation, liposome-mediated transfection and transduction using retrovirus or other virus, e.g. adenovirus, AAV, lentivirus or vaccinia. For bacterial cells, suitable techniques may include calcium chloride transformation, electroporation and transfection using bacteriophage.

Marker genes such as antibiotic resistance or sensitivity genes may be used in identifying clones containing nucleic acid of interest, as is well-known in the art.

The introduced nucleic acid may be on an extra-chromosomal vector within the cell or the nucleic acid may be integrated into the genome of the host cell. Integration may be promoted by inclusion of sequences within the nucleic acid or vector which promote recombination with the genome, in accordance with standard techniques.

In some disclosure herein, nucleic acid encoding a modified serpin as described herein may be contained in a vector suitable for administration to an individual e.g. for gene therapy applications. Suitable vectors include retroviral vectors, lentiviral vectors, adenoviral vectors and AAT vectors.

The introduction may be followed by expression of the nucleic acid to produce the encoded modified serpin. In some disclosure herein, host cells (which may include cells actually transformed although more likely the cells will be descendants of the transformed cells) may be cultured *in vitro* under conditions for expression of the nucleic acid, so that the encoded serpin polypeptide is produced. When an inducible promoter is used, expression may require the activation of the inducible promoter.

The expressed polypeptide comprising or consisting of the modified serpin may be isolated and/or purified, after production. This may be achieved using any convenient method known in the art. Techniques for the purification of recombinant polypeptides are well known in the art and include, for example HPLC, FPLC or affinity chromatography. In some disclosure herein, purification may be performed using an affinity tag on the polypeptide as described above.

Other disclosure herein provides a method of producing a modified serpin comprising expressing a nucleic acid encoding a modified serpin as described above in a host cell and optionally isolating and/or purifying the modified serpin thus produced.

Polypeptides comprising or consisting of a modified serpin produced as described may be investigated further, for example the pharmacological properties and/or activity may be determined. Methods and means of protein analysis are well-known in the art.

A modified serpin as described herein, nucleic acid encoding a modified serpin or a recombinant cell expressing a modified serpin, may be useful in therapy. For example, a modified serpin as described herein, nucleic acid encoding a modified serpin or a recombinant cell expressing a modified serpin may be administered to an individual for the treatment of bleeding.

Whilst a modified serpin may be administered alone, modified serpins will usually be administered in the form of a pharmaceutical composition, which may comprise at least one component in addition to the modified serpin e.g. a nucleic acid encoding the modified serpin or recombinant cell expressing the modified serpin. Thus pharmaceutical compositions may comprise, in addition to the modified serpin, nucleic acid or cell, a pharmaceutically acceptable excipient, carrier, buffer, stabilizer or other materials well known to those skilled in the art. The term "pharmaceutically acceptable" as used herein pertains to compounds, materials, compositions, and/or dosage forms which are, within the scope of sound medical judgement, suitable for use in contact with the tissues of a subject (e.g., human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. The precise nature of the carrier or other material will depend on the route of administration, which may be by bolus, infusion, injection or any other suitable route, as discussed below.

In some disclosure herein, modified serpins, nucleic acids or cells may be provided in a lyophilised form for reconstitution prior to administration. For example, lyophilised serpins may be re-constituted in sterile water and mixed with saline prior to administration to an individual.

For parenteral, for example sub-cutaneous or intra-venous administration, e.g. by injection, the pharmaceutical composition comprising the modified serpin, nucleic acid or cell may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or nonionic surfactants, such as TWEEN™, PLURONICS™ or polyethylene glycol (PEG). Suitable carriers, excipients, etc. can be found in standard pharmaceutical texts, for example, Remington's Pharmaceutical Sciences, 18th edition, Mack Publishing Company, Easton, Pa., 1990.

Pharmaceutical compositions and formulations may conveniently be presented in unit dosage form and may be prepared by any methods well known in the art of pharmacy. Such methods include the step of bringing into association the modified serpin with the carrier which constitutes one or more accessory ingredients. In general, the compositions are prepared by uniformly and intimately bringing into association the active compound with liquid carriers or finely divided solid carriers or both, and then if necessary shaping the product.

Preferably, modified serpins, nucleic acids or cells as described herein are formulated in a pharmaceutical composition for intra-venous or sub-cutaneous administration.

A pharmaceutical composition comprising a modified serpin, nucleic acid or cell may be administered alone or in combination with other treatments, either simultaneously or sequentially dependent upon the condition to be treated.

A modified serpin, nucleic acid or cell as described herein may be used in a method of treatment of the human or animal body, including therapeutic and prophylactic or preventative treatment (e.g. treatment before the onset of a condition in an individual to reduce the risk of the condition occurring in the individual; delay its onset; or reduce its severity after onset). The method of treatment may comprise administering a modified serpin to an individual in need thereof.

An individual suitable for treatment as described above may be a mammal, such as a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orang-utan, gibbon), or a human.

In some disclosure herein, the individual is a human. In other disclosure herein, non-human mammals, especially mammals that are conventionally used as models for demonstrating therapeutic efficacy in humans (e.g. murine, primate, porcine, canine, or rabbit animals) may be employed. The inhibition of human and murine APC by modified serpins without the inhibition of human or murine thrombin is shown below.

Administration is normally in a "therapeutically effective amount" or "prophylactically effective amount", this being sufficient to show benefit to a patient. Such benefit may be at least amelioration of at least one symptom. The actual amount administered, and rate and time-course of administration, will depend on the nature and severity of what is being treated, the particular mammal being treated, the clinical condition of the individual patient, the cause of the disorder, the site of delivery of the composition, the method of administration, the scheduling of administration and other factors known to medical practitioners.

A composition may be administered alone or in combination with other treatments, either simultaneously or sequentially dependent upon the circumstances of the individual to be treated.

Prescription of treatment, e.g. decisions on dosage etc, is within the responsibility of general practitioners and other medical doctors and may depend on the severity of the symptoms and/or progression of a disease being treated. Appropriate doses of therapeutic polypeptides are well known in the art (Ledermann J.A. et al. (1991) Int. J. Cancer 47: 659-664; Bagshawe K.D. et al. (1991) Antibody, Immunoconjugates and Radiopharmaceuticals 4: 915-922). Specific dosages may be indicated herein or in the Physician's Desk Reference (2003) as appropriate for the type of medicament being administered may be used. A therapeutically effective amount or suitable dose of a modified serpin may be determined by comparing its *in vitro* activity and *in vivo* activity in an animal model. Methods for extrapolation of effective dosages in mice and other test animals to humans are known. The precise dose will depend upon a number of factors, including whether the modified serpin is for prevention or for treatment, the size and location of the area to be treated, the precise nature of the modified serpin and the nature of any detectable label or other molecule attached to the modified serpin.

A typical modified serpin dose will be in the range of 0.1 mg/kg to 100mg/kg, for example 1 to 80mg/kg. For example, a dose in the range 100 µg to 1 g may be used for systemic applications, and 1 µg to 1 mg for topical applications. An initial higher loading dose, followed by one or more lower doses, may be administered. This is a dose for a single treatment of an adult patient, which may be proportionally adjusted for children and infants, and also adjusted for other modified serpin formats in proportion to molecular weight. Treatments may be repeated at daily, twice-weekly, weekly or monthly intervals, at the discretion of the physician. The treatment schedule for an individual may be dependent on the pharmocokinetic and pharmacodynamic properties of the modified serpin composition, the route of administration and the nature of the condition being treated.

Treatment may be periodic, and the period between administrations may be about one week or more, e.g. about two weeks or more, about three weeks or more, about four weeks or more, about once a month or more, about five weeks or more, or about six weeks or more. For example, treatment may be every two to four weeks or every four to eight weeks. Treatment may be given before, and/or after surgery, and/or may be administered or applied directly at the anatomical site of trauma, surgical treatment or invasive procedure. Suitable formulations and routes of administration are described above.

In some disclosure herein, modified serpins as described herein may be administered as sub-cutaneous injections. Sub-cutaneous injections may be administered using an auto-injector, for example for long term prophylaxis/treatment.

In some disclosure herein, the therapeutic effect of the modified serpin may persist for several half-lives, depending on the dose.

Modified serpins described herein inhibit APC without inhibiting or substantially inhibiting procoagulant factors, such as thrombin, and may be useful in the treatment of bleeding and bleeding disorders; in particular disorders caused by reduced thrombin generation or increased APC anticoagulant pathway activity.

Hemostasis is the normal coagulation response to injury i.e. the prevention of bleeding or hemorrhage, for example from a damaged blood vessel. Hemostasis arrests bleeding and hemorrhage from blood vessels in the body. Modified serpins may promote hemostasis i.e. they may promote or increase the arrest of bleeding and hemorrhage from blood vessels in the body, for example in individuals with bleeding disorders or trauma.

The disclosure provides; a modified serpin as described herein for use in a method of treatment of the human or animal body; a modified serpin as described herein for use in a method of treatment of bleeding or the promotion of hemostasis; the use of a modified serpin as described herein in the manufacture of a medicament for the treatment of bleeding or the promotion of hemostasis; and a method of treatment of bleeding or the promotion of hemostasis comprising administering a modified serpin as described herein to an individual in need thereof.

Bleeding may include bleeding or hemorrhage from blood vessels in the body.

An individual suitable for treatment with a modified serpin as described herein may have a bleeding disorder.

Bleeding disorders may be caused or associated with reduced thrombin generation or increased activity of the APC anticoagulant pathway.

Bleeding disorders may include any heritable or acquired bleeding disorder in which there is reduced thrombin generation, reduced fibrin clot formation or reduced clot stability. For example, bleeding disorders may include congenital deficiencies of factors VII, VIII, X, IX, XI and XIII; combined V and VIII deficiency; prothrombin deficiency; fibrin deficiency; and rare deficiencies of other clotting factors; hemophilia A, B and C; increased bleeding associated with hyperfibrinolysis; increased bleeding due to reduced platelet count or reduced platelet function; and von Willebrand Disease.

Acquired bleeding disorders may include dilutional coagulopathy associated with major blood loss, bleeding resulting from trauma and surgery and the effects of anticoagulant therapy.

In some disclosure herein, the individual may be resistant to exogenous coagulation factors, such as exogenous fVIII or fIX. For example, exogenous fVIII or fIX may elicit an immune response in the individual, for example the production of inhibitory alloantibodies.

An individual suitable for treatment with a modified serpin as described herein may have an acquired bleeding disorder, such as bleeding related to trauma, surgery or anti-coagulant therapy. For example, an individual suitable for treatment with a modified serpin as described herein may have suffered a trauma; or may have undergone or be undergoing surgery or anti-coagulant therapy. Suitable individuals may be bleeding or at risk of bleeding from one or more blood vessels in the body.

In some disclosure herein, a modified serpin as described herein may be useful in the prevention or treatment of i) bleeding in patients with clotting factor alloantibodies; ii) bleeding in patients at high risk of inhibitor development, for example to avoid development of alloantibodies; iii) bleeding in patients with factor VIII deficiency in the absence of inhibitors; iv) bleeding in patients with congenital bleeding disorders, for example a congenital bleeding disorder for which there is no current recombinant optimal replacement therapy, such as severe factor VII deficiency, factor XI deficiency, combined VIII & V deficiency, factor X deficiency and factor V deficiency; v) bleeding in patients with hemophilia, for example patients for whom replacement therapy is inappropriate or unavailable; or vi) acquired bleeding, including bleeding related to trauma, surgery, and anticoagulant therapy.

Other disclosure herein provides the use of a modified serpin as described herein as a procoagulant and the use of a modified serpin to inhibit APC in the treatment of bleeding.

Various further aspects and embodiments of the present invention will be apparent to those skilled in the art in view of the present disclosure.

Other aspects and embodiments of the invention provide the aspects and embodiments described above with the term "comprising" replaced by the term "consisting of" and the aspects and embodiments described above with the term "comprising" replaced by the term "consisting essentially of".

It is to be understood that the application discloses all combinations of any of the above aspects and embodiments described above with each other, unless the context demands otherwise. Similarly, the application discloses all combinations of the preferred and/or optional features either singly or together with any of the other aspects, unless the context demands otherwise.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Certain aspects and embodiments of the invention will now be illustrated by way of example and with reference to the figures described above and the tables described below.

Table 1 shows second-order rate constants for the inhibition of thrombin and APC by A22 PCI (PCI with an N-terminal truncation, starting at Ala22, numbering uses the mature protein sequence, including the propeptide), FL α₁AT Pitts (full length α₁AT with the P1R Pittsburgh (Pitts) mutation) and their variants. Standard errors are shown. * The rate constant of inhibition for thrombin by the P2KP1' variant of PCI is an estimate as after initial inhibition, reactions do not appear to approach complete inhibition, potentially due to serpin being shuttled down the substrate pathway or dissociation of the covalent serpin:protease inhibitory complex

Table 2 shows second-order rate constants for the inhibition of thrombin and APC by PCI and variants in the presence of heparin. Standard errors are shown. * the rate constant of inhibition for thrombin by the P2KP1'K variant of PCI is an estimate from the initial slope of the plot of residual thrombin activity versus time. Complete inhibition is not achieved, potentially because of serpin being shuttled down the substrate pathway or dissociation of the covalent serpin:protease inhibitory complex.

Table 3 shows second-order rate constants for the inhibition of fXa by α₁AT Pitts and PCI and their variants. Standard errors are shown.

Table 4 shows second-order rate constants for the inhibition of fXIa by PCI and the PCI P2KP1'K variant. APC inhibition is shown for comparison (from Table 1). Standard errors are shown.

Table 5 shows second-order rate constants for the inhibition of thrombin and APC by PCI variants generated by targeted random mutagenesis. Standard errors are shown. Constants for WT and P2KP1' PCI are shown for comparison. * The rate constant of inhibition for thrombin by the P2KP1' variant of PCI is an estimate as after initial inhibition, reactions do not appear to approach complete inhibition, potentially due to serpin being shuttled down the substrate pathway or dissociation of the covalent serpin:protease inhibitory complex.

Table 6 shows second-order rate constants for the inhibition of fXIa by FL α₁AT Pitts C232S and its P2KP1'K variant. APC inhibition is shown for comparison (from Table 1). Standard errors are shown.

Table 7 shows a fraction of the RCL sequences of the PCI variants determined by targeted random mutagenesis to be specific for APC inhibition over thrombin inhibition. Sequences shown are from an initial experiment in which 88 mutants were assessed. The P4, P2 and P1' residues that were varied in this experiment are shown in bold. WT PCI and P2KP1'K PCI sequences are shown for comparison.

Table 8 shows a fraction of the RCL sequences of the PCI variants determined by targeted random mutagenesis to be specific for APC inhibition over thrombin inhibition. Sequences shown are from a larger experiment in which 460 mutants were assessed. The P4, P2 and P1' residues that were varied in this experiment are shown in bold. WT PCI and P2KP1'K PCI sequences are shown for comparison.

Table 9 shows a fraction of the RCL sequences of the α₁AT variants determined by targeted random mutagenesis to be specific for APC inhibition over thrombin inhibition. Sequences are shown compared to both WT α₁AT and α₁AT Pitts. The P2 and P1' residues are shown in bold, the P1 residues are underlined. Prefixes show the library of origin for the particular mutant with mutants denoted P2.nr coming from the P2 variant library P1'.nr. coming from the P1' variant library and mutants labelled 1-5.nr. coming from plates 1-5 of the P2P1' variant library.

Table 10 shows second-order rate constants for the inhibition of thrombin and APC by a subset of variants of α₁AT determined by targeted random mutagenesis to be more specific for APC than for thrombin. Standard errors are given. The second-order rate constants for thrombin and APC inhibition by FL α₁AT Pitts C232S P2KP1'K and FL α₁AT Pitts C232S are given for comparison.

Table 11 shows the results of PT and aPTT assays to investigate inhibition of procoagulant proteases by hits from random mutagenesis on the FL α₁AT Pitts C232S background. PT assays were performed using 1/4 diluted plasma to increase the sensitivity of the assay and performed in triplicate, except for the reactions shown for FL α₁AT Pitts C232S P2RP1'C, which were performed in duplicate. The error shown is the standard deviation. aPTT assays were single experiments, no error is shown. For both PT and aPTT a buffer only control, where instead of protein, buffer (TBS) was added to the plasma was used as a control. Increases in PT or aPTT with respect to the control are an indication of inhibition of procoagulant proteases. For both PT and aPTT assays, the serpin mutants were used at a 5 µM concentration. For comparison, the P2K and P1'K mutants are shown as an average of triplicates. As shown from inhibition rate constants in Tables 1 and 3, these mutants show high specificity for APC over thrombin, but inhibit fXa significantly. They are therefore good comparator for an inhibition of procoagulant proteases other than thrombin. ND indicates not determined.

Table 12 shows second-order rate constants for the inhibition of fXa by α₁AT variants from targeted random mutagenesis. The mutants evaluated here showed specificity for APC over thrombin, substantial APC inhibition and showed no prolongation of the PT. Most also showed only minor aPTT prolongation. Because of these features, they were selected for further analysis. For comparison, fXa inhibition by FL α₁AT Pitts C232S and FL α₁AT Pitts C232S P2KP1'K is also shown (from Table 3).

Table 13 shows a summary of the characterization of two more mutants of α₁AT found by combining information from rational and random mutagenesis. FL α₁AT Pitts C232S and FL α₁AT Pitts C232S P2KP1'K C232S are shown for comparison (data from Tables 1 and 3 and Figure 5). aPTTs for P2RP1'Q and P2KP1'Q are the average of four separate measurements, the error shown is the standard deviation. The value obtained for plasma with buffer is shown in brackets, again with the standard deviation shown as the error. The aPTTs shown for Pitts and P2KP1'K are the result of at least three separate measurements, the error shown is the standard deviation. The value shown in brackets is the value obtained for plasma with buffer, shown with the standard deviation as the error. All aPTTs were obtained using a final concentration of 5 µM serpin. FL α₁AT Pitts C232S at this concentration rendered the plasma unclottable. The value of 300 s shown is the cut-off for the assay. Second-order rate constants for the inhibition of thrombin, APC and fXa by the variants are shown with the standard error.

### Experiments

The coagulation cascade and the regulatory role of serpins in this cascade are shown in Figure 1. Two pathways lead to activation of the coagulation cascade, the extrinsic cascade (or tissue factor pathway) and the intrinsic pathway (contact activation pathway). The main physiological pathway of activation is believed to be the extrinsic pathway. In this pathway, tissue factor (TF) is exposed on the surface of the damage blood vessel. TF can then bind fVIIa and fVII. TF:fVIIa activates fVII, as well as TF:fVII spontaneously activating to TF:fVIIa. TF:fVIIa activates fX to fXa and this activates prothrombin to thrombin (fIIa); the central protease of the coagulation cascade. Thrombin activates platelets by cleavage of protease activated receptors (PARs) and cleaves fibrinogen to fibrin. Fibrin is crosslinked by fXIIIa, which is itself activated by thrombin, to form the stable fibrin clot. Thrombin in addition activates a positive feedback mechanism to potentiate its own formation. It activates fVIII to fVIIIa and fV to fVa. fVIIIa binds to fIXa to form the intrinsic tenase (Xase) complex. The intrinsic Xase activates more fX. This fXa can bind to fVa to form prothrombinase. Prothrombinase activates prothrombin to thrombin and is responsible for most of the thrombin generated after initiation of coagulation. In addition to thrombin's positive feedback mechanism, thrombin can also shut down its own activation via a negative feedback mechanism. When it binds its cofactor thrombomodulin (TM), the thrombin:TM complex can activate protein C (PC) to activated protein C (APC). APC cleaves and inactivates both fVIIIa and fVa, effectively shutting down thrombin generation. Serpins are important inhibitors of the coagulation cascade. The inhibitory actions of the serpins protein C inhibitor (PCI), antithrombin (ATIII), heparin cofactor II (HCII) and α₁-antitrypsin (α₁AT) are shown in Figure 1.

Below we describe the conversion of serpins into specific inhibitors of APC for use as procoagulant agents (treatment, prophylaxis, bypassing or synergistic) in the treatment of bleeding disorders such as hemophilia. The modifications described here for both PCI and α₁AT show as a proof of principle that small changes in these proteins can be used to create specific APC inhibitors.

PCI was first described as the physiological inhibitor of APC and therefore served as a starting point for these investigations (Suzuki *et al,* 1983; 1984). However, PCI is promiscuous and also inhibits thrombin, thrombin:TM, fXIa, fXa and TF:fVIIa (Elisen *et al,* 1998; Mosnier *et al,* 2001; Suzuki *et al,* 1984; Fortenberry *et al,* 2011; Meijers *et al,* 1988). As a consequence, PCI can function as both a pro- and an anticoagulant. Its activity is regulated by binding to glycosaminoglycans, such as heparin and heparan sulfates (Pratt & Church, 1992; Pratt *et al,* 1992; Li & Huntington, 2008). For an overview of the role of PCI in the coagulation cascade, see Figure 1.

α₁AT is the natural inhibitor of neutrophil elastase (Kalsheker, 1989). Unlike other serpins in the coagulation cascade that have either Arg or Leu at P1, α₁AT has a Met instead. This makes it a very poor inhibitor of coagulation proteases. Nevertheless, because of the high concentration of α₁AT in plasma, it is believed to inhibit APC to a physiologically significant degree (Heeb & Griffin, 1988). Mutagenesis of the P1 residue has shown that the use of an Arg or Leu at P1 drastically improves inhibition of coagulation proteases by α₁AT (Heeb *et al,* 1990). This is exemplified by the Pittsburgh (M358R (P1R); Pitts) variant of α₁AT that causes a severe bleeding disorder (Owen *et al,* 1983).

To develop serpins specific for inhibition of APC over procoagulant proteases, PCI and α₁AT Pittsburgh (P1R, α₁AT Pitts) were used as template serpin scaffolds. All proteins used in this study were expressed from *Escherichia coli* cultures (Rosetta2(DE3)pLysS, Novagen) using the pETSUMO expression vector (Invitrogen) and purified using a combination of Ni-chromatography, anion-exchange and in the case of PCI, heparin affinity chromatography. The SUMO-tag was subsequently removed by SUMO protease cleavage and the tag removed by tandem Ni-anion exchange chromatography for α₁AT Pittsburgh (Pitts, P1R) and tandem anion exchange-heparin chromatography for PCI. The PCI construct is N-terminally truncated, starting at Ala22 (A22, numbering according to the mature protein sequence, starting with the propeptide). The α₁AT Pitts (P1R) construct is full-length (FL) and has an additional C232S mutation to abolish intermolecular disulfide bond formation and other modifications during expression and purification (Yamasaki et *al,* 2011). Due to the expression vector used, the α₁AT Pitts construct has a Ser (S) as its first residue instead of a Glu (E). The C232S and E1S mutations are not expected to alter the activity of α₁AT.

Lys residues were introduced at different positions into the RCL of PCI and α₁AT Pittsburgh and the resulting mutants tested for inhibition of thrombin and APC. In the initial stages of this study, inhibitors were not screened or tested for their inhibition of other coagulation proteases on the premise that once thrombin inhibition was abolished in favor of APC inhibition, the inhibitor could potentially be additionally modified if it had significant residual inhibitory activity towards other coagulation proteases. The RCL residues are numbered according to the Schechter-Berger nomenclature for serpins (Schechter & Berger, 1967).

Rate constants of thrombin and APC inhibition were measured under pseudo-first-order conditions using an excess of serpin over protease (Table 1). Serpin and protease were incubated together for varying lengths of time and the residual activity was determined by adding an excess of chromogenic substrate for the protease (S2238 for thrombin and S2366 for APC). Residual protease activity was then measured by following the absorbance at 405 nm. Plots of residual protease activity over time gave the observed rate constant k_{obs}. The second-order rate constant, k₂, is the slope of the plot of k_{obs} versus serpin concentration (fitted using a linear regression model). Standard errors of the slope are shown.

The lysine mutations introduced at P2 and P1' were highly effective at increasing the specificity of PCI and α₁AT for APC over thrombin for all variants shown in Table 1. Generally, thrombin inhibition was greatly reduced in all cases. APC inhibition was also reduced for all mutants but not nearly to the same degree. Both serpins initially inhibited thrombin better than APC. This was reversed for all mutants tested.

PCI, unlike α₁AT, binds heparin and this binding considerably increases its inhibition of thrombin and APC (Pratt & Church, 1992). We therefore tested the inhibition of thrombin and APC by the P1'K and P2KP1'K PCI mutants in the presence of heparin to see if the swap in specificity seen in Table 1 would persist. Rate constants were measured under pseudo-first-order conditions using an excess of PCI over protease. PCI was preincubated with an equimolar concentration of unfractionated heparin for 30 min prior to the experiment. PCI:heparin and protease were incubated together for varying lengths of time and the residual activity after certain timepoints determined by adding an excess of chromogenic substrate for the protease mixed with polybrene to bind the heparin. Plots of residual protease activity over time gave the observed rate constant k_{obs}. The second-order rate constant, k₂, is the slope of the plot of k_{obs} versus serpin concentration (fitted using a linear regression model). Standard errors of the slope are shown. The value calculated for the inhibition of thrombin by P2KP1'K PCI was an estimate from the initial slope of the plot of residual thrombin activity versus time, as the graph suggests that complete inhibition is not achieved. This might be due to the substrate pathway or complex dissociation. The second-order rate constants are shown in Table 2. As for the inhibition in the absence of heparin, the P1'K and P2KP1'K mutants of PCI, unlike the WT protein were specific for APC over thrombin (Table 2).

These experiments showed that introducing only one or two modifications in the serpin RCL was sufficient to abolish or greatly reduce the inhibition of thrombin both in the presence and absence of cofactors. The inhibition of APC was reduced but still considerable, especially for the variants of α₁AT and the PCI variants in the presence of heparin. However, the specificity of PCI and α₁AT Pitts is not limited to thrombin and APC. Both these serpins also inhibit fXa, another procoagulant protease. In order not to inhibit coagulation, our variants also need to be specific for APC over fXa. We therefore also determined the rate constants of inhibition of PCI and α₁AT and their variants for fXa (Table 3) . Rate constants were measured under pseudo-first-order conditions using an excess of serpin over protease. Serpin and protease were incubated together for varying lengths of time and the residual activity determined by adding an excess of chromogenic substrate (S2222) for the protease. Plots of residual protease activity over time gave the observed rate constant k_{obs}. The second-order rate constant, k₂, is the slope of the plot of k_{obs} versus serpin concentration (fitted using a linear regression model). Standard errors of the slope are shown (Table 3).

As seen before for thrombin, WT PCI inhibited fXa better than APC. α₁AT Pitts inhibited APC better than fXa, but the inhibition of fXa was still considerable. The inhibition of fXa was still significant for P1'K PCI, P2K α₁AT Pitts and P1'K α₁AT Pitts (Table 3). The P2KP1'K variants of α₁AT Pitts and PCI were both highly specific for APC over fXa, with absent or negligible inhibition of fXa and were therefore considered lead candidates. The P1'K variant of PCI is also of interest as its inhibition of fXa is very slow in the absence of heparin. The presence of heparin accelerates the rate of APC inhibition significantly, which could potentially skew the specificity ratio in favor of APC.

The PCI lead compounds will be discussed first, followed by the α₁AT lead compound.

To investigate the properties of A22 P2KP1'K PCI in a more complex plasma system and to rule out any negative effects on the procoagulant proteases, a prothrombin time assay (PT) was performed. This assay measures the time until clot formation in plasma after coagulation is initiated via the extrinsic pathway. A22 WT PCI showed a small increase in the clotting time, whereas P2KP1'K showed a smaller increase, consistent with reduced inhibitory activity towards procoagulant proteases (Figure 2).

In addition, we wanted to rule out any effect of the PCI mutant on the contact activation pathway of coagulation. To do so, rate constants of inhibition were measured for the inhibition of fXIa and an aPTT assay was done. This assay is similar to the PT assay except that it measures coagulation initiated via the intrinsic pathway.

Second-order rate constants of inhibition for inhibition of fXIa by PCI and the P2KP1'K variant were measured under pseudo-first-order conditions using an excess of serpin over protease. Serpin and protease were incubated together for varying lengths of time and the residual activity determined by adding an excess of chromogenic substrate for the protease (S2366). Plots of residual protease activity over time gave the observed rate constant k_{obs}. The second-order rate constant, k₂, is the slope of the plot of k_{obs} versus serpin concentration (fitted using a linear regression model). Standard errors of the slope are given. fXIa inhibition by A22 P2KP1'K PCI did not go to completion over the course of the experiment, potentially due to serpin cleavage by the protease. Compared to WT, the P2KP1'K mutant showed a much reduced inhibition towards fXIa and greater specificity for APC than for fXIa (Table 4) .

The aPTT assay showed that WT PCI was a potent inhibitor of the contact activation pathway, potentially due to inhibition of fXIa or fXIIa (Figure 3). The P2KP1'K mutant showed a small increase in the aPTT. However, since the contact activation pathway primarily activates coagulation through fIXa activation, inhibition of contact activation to a small extent is likely to be insignificant in hemophiliacs as they are deficient in either the main target of contact activation (fIX) or its cofactor (fVIII).

The results shown here so far therefore show that both A22 P1'K PCI and A22 P2KP1'K PCI are promising, APC-specific lead compounds for development into bypassing agents for the treatment of hemophilia.

To generate additional PCI mutants with specificity for APC over thrombin, a targeted random mutagenesis strategy was employed on the PCI scaffold. The residues targeted were P4, P2 and P1'. The random approach is based on a selection for APC inhibition and against thrombin inhibition by testing inhibitory activity of bacterial lysates, after PCI expression in a 96-well format.

The assay was calibrated using the most specific PCI mutant generated from the rational mutagenesis combined with the testing for specificity outlined above; A22 P2KP1'K PCI. WT PCI was used as an additional control. The negative selection against inhibition of thrombin was achieved by incubating bacterial lysates for a time period such that A22 WT PCI showed complete inhibition and the incubation period extended from that timepoint on to also select against minor inhibition. Positive selection for APC inhibition was calibrated such that both WT and P2KP1'K PCI fell into the intermediate range of APC inhibitory activity so we would be able to determine both increases as well as decreases in inhibition.

In an initial assay, 88 variants were screened for thrombin and APC inhibitory activity. Cultures were grown, induced and protein expressed in 96-well plates. Cells were lysed by the addition of a lysis buffer and the lysates assayed for inhibitory activity against thrombin and APC by incubating the lysate with the protease for 1 h for thrombin and 30 min for APC. Residual protease activity was then read by addition of a chromogenic substrate to the protease. A22 WT PCI and A22 P2KP1'K PCI were used as controls. Any lysate with higher or equivalent residual thrombin activity and lower or equivalent residual APC activity compared to P2KP1'K PCI was considered to be a promising APC-specific candidate. These sequences are shown in Table 7. P4, P2 and P1' positions are shown in bold.

The mutant with the greatest APC inhibitory activity from this set of experiments (D8; P4QP2RP1'N) was characterized in a preliminary fashion and was shown to be more specific for the inhibition of APC than thrombin, whilst utilizing different mutations than P2KP1'K (Table 5). This indicated that it would be possible to make additional mutations in the serpin RCL, which would have equivalent effects to the mutations already described.

To generate a larger dataset, a further 460 mutants were screened in both the positive and negative selection assays. Cultures were grown, induced and protein expressed in five 96-well plates. Cells were lysed by the addition of a lysis buffer and the lysates assayed for inhibitory activity against thrombin and APC by incubating the lysate with the protease for 1 h for thrombin and 30 min for APC. Residual protease activity was then read by addition of a chromogenic substrate to the protease (S2238 for thrombin, S2366 for APC). A22 WT PCI and A22 P2KP1'K PCI were used as controls. Any lysate with higher or equivalent residual thrombin activity and lower or equivalent residual APC activity compared to P2KP1'K PCI was considered to be a promising APC-specific candidate. From this dataset, colonies were picked and sequenced for serpins that showed better inhibition of APC and worse or equivalent inhibition of thrombin than P2KP1'K and these were retested in triplicate in the same assay to verify that these were indeed true hits and not false positives. From this retest a set of 15 out of the 17 mutants found in the initial screen was determined to be better or equivalent at inhibiting APC than P2KP1'K and worse or equivalent at inhibiting thrombin (Table 8). Sequences of variants that were positive after retesting are shown in Table 8. Interestingly, the random mutagenesis confirmed the beneficial effects of positively charged residues in the P2 and P1' positions. However alternative RCL compositions were also found.

Preliminary second-order rate constants for the inhibition of thrombin and APC by random mutagenesis PCI variants were measured under pseudo first-order conditions using an excess of serpin over protease. Serpin and protease were incubated together for varying lengths of time and the residual activity determined by adding an excess of chromogenic substrate for the protease. Plots of residual protease activity over time gave the observed rate constant k_{obs}. The second-order rate constant, k₂, is the slope of the plot of k_{obs} versus serpin concentration (fitted using a linear regression model). WT and P2KP1'K are shown for comparison. Rate constants are shown in Table 5. The error shown is the standard error of the slope.

Preliminary characterization of some of the selected mutants from the random mutagenesis assay showed that the selected mutants were at least functionally equivalent to P2KP1'K PCI, and some had a slightly improved rate of APC inhibition (Table 5). These experiments strongly suggest that P2KP1'K is not the only composition which could be utilized to generate APC-specific serpins.

The main lead compound based on α₁AT Pitts from the rate constants shown in Tables 1 and 3 was FL α₁AT Pitts C232S P2KP1'K. This mutant was shown not to inhibit thrombin and only slowly inhibited fXa, but retained APC inhibition (Tables 1 and 3).

To investigate the properties of this mutant in a more complex plasma system and to rule out any negative effects on the procoagulant proteases, a prothrombin time assay (PT) was performed. This assay measures the time until clot formation after coagulation is initiated via the extrinsic pathway. As expected, the anticoagulant α₁AT Pitts showed an increase in clotting time, due to its inhibition of thrombin and fXa (Figure 4). In contrast, the P2KP1'K mutant of α₁AT Pitts showed no increase in clotting time and therefore did not interfere with normal coagulation in this assay.

The data so far indicated that the P2KP1'K mutant of α₁AT Pitts did not interfere with procoagulant proteases in either the extrinsic (tissue factor) pathway or the common pathway of coagulation. In addition we wanted to determine whether FL α₁AT Pitts C232S P2KP1'K affected the intrinsic (contact activation) pathway. Second-order rate constants of inhibition of fXIa by FL α₁AT Pitts C232S and the P2KP1'K variant were measured under pseudo first-order conditions using an excess of serpin over protease. Serpin and protease were incubated together for varying lengths of time and the residual activity determined by adding an excess of chromogenic substrate for the protease (S2366). Plots of residual protease activity over time gave the observed rate constant k_{obs}. The second-order rate constant, k₂, is the slope of the plot of k_{obs} versus serpin concentration

(fitted using a linear regression model). Standard errors of the slope are given.

fXI is activated during the contact activation pathway and it feeds into the common pathway of coagulation by activating fIX. In addition, fXI is activated once coagulation is initiated by thrombin. fXIa was inhibited to a significant degree by FL α₁AT Pitts C232S, however this inhibition was greatly reduced for FL α₁AT Pitts C232S P2KP1'K (Table 6).

Because a small degree of inhibition of fXIa by the P2KP1'K mutant of α₁AT Pitts was detected and to determine any potential negative effect on fXIIa, we additionally performed an aPTT assay. This assay is similar to the PT assay except that it measures coagulation initiated via the intrinsic pathway of coagulation. The aPTT could therefore be used to detect any negative effect on fXIa and the contact activation pathway of coagulation. In this assay, plasma incubated with FL α₁AT Pitts C232S did not clot within the time of the assay except for one reaction with 0.67 µM serpin (Figure 5A). FL α₁AT Pitts C232S P2KP1'K showed a small increase in clotting time, but there was no dose-dependent increase (Figure 5B). This indicates that the fXIa inhibitory activity of FL α₁AT Pitts C232S P2KP1'K is likely too slow to significantly affect the contact activation pathway. In addition, the contact activation pathway activates the coagulation cascade via activation of fIXa. Since hemophiliacs lack either fIX or its essential cofactor fVIII, the role of a small degree of inhibition of the contact activation pathway in hemophiliacs is likely to be minimal.

To investigate whether the P2KP1'K mutant of α₁AT Pitts was able to inhibit APC in a plasma system, we used a modified thrombin generation assay (TGA). Thrombin generation was measured in human pooled normal plasma (NP) in the presence and absence of recombinant soluble thrombomodulin (TM). This TM was expressed and purified from a HEK-EBNA expression system and comprises the soluble extracellular domain. TM is not normally present in the TGA because physiologically it is a transmembrane protein, present on the endothelial membrane and largely absent from plasma. Therefore, there is no activation of the PC pathway in a normal TGA or other coagulation assays utilizing plasma, such as the PT and aPTT assays. Adding TM to the assay allows PC activation and thereby might give a more realistic picture of thrombin generation *in vivo.* Assays shown in Figures 6 and 7 were performed in pooled normal human plasma (NP) from George King Biomedical. Coagulation was initiated by the addition of CaCl₂ and TF/phospholipid (RB low TF and phospholipid reagent, Technoclone) to activate coagulation through the extrinsic pathway. Thrombin generation was measured through the cleavage of a fluorogenic substrate (Z-Gly-Gly-Arg-AMC). Fluorescence units were converted to thrombin concentration by calibrating fluorescence units against known concentrations of thrombin, using the Technothrombin calibration kit (Technoclone).

Addition of TM to pooled normal plasma reduced thrombin generation in a concentration-dependent manner. From this experiment we chose two concentrations of TM to knock down thrombin generation to either intermediate levels (1.25 nM TM final assay concentration) or to low levels (10 nM TM final assay concentration). These concentrations were used in subsequent assays to evaluate the ability of FL α₁AT Pitts C232S P2KP1'K to inhibit APC in plasma.

Addition of FL α₁AT Pitts C232S to normal human plasma (NP) reduced thrombin generation at all concentrations used, likely due to the inhibition of thrombin as well as fXa (Figure 6). In contrast, FL α₁AT Pitts C232S P2KP1'K had no effect on NP in the absence of TM (Figure 7A). However in the presence of TM, FL α₁AT Pitts C232S P2KP1'K dose-dependently rescued thrombin generation (Figure 7B-D). This effect is the result of specific inhibition of APC by FL α₁AT Pitts C232S P2KP1'K.

In order to perform the same experiments in fVIII- or fIX-deficient plasma, it was necessary to increase the amount of tissue factor (TF) used to initiate the assay because at the baseline conditions (RB trigger only), there was no detectable thrombin generation in factor deficient plasma. To demonstrate the effect of an increase in TF on thrombin generation, the reactions were spiked with different dilutions of TF reagent (Dade Innovin, Siemens) in addition to the RB reagent used to trigger the assay at baseline conditions. The concentration of TF in the Innovin reagent is not disclosed by the manufacturer, however previous measurements have shown it to be around 7.36 nM (Duckers *et al,* 2010). Increasing the TF trigger shortened lag-time and increased both peak thrombin and endogenous thrombin potential (ETP) in human NP, fVIII-deficient (HA) and fIX-deficient (HB) plasma. From these experiments we chose an Innovin dilution of 1:4,000 in the final reaction to initiate thrombin generation. RB reagent, which contains both phospholipids and TF was added because of the need to add phospholipids to the assay.

Because the use of factor-deficient plasma required modification of the assay parameters, we repeated the TM titration experiment for human normal pooled plasma in human HA plasma with the addition of 1:4,000 Innovin. The assay was performed in human fVIII-deficient plasma (less than 1% fVIII activity) from George King Biomedical. Coagulation was initiated by the addition of CaCl₂ and/or TF/phospholipid (RB low TF and phospholipid reagent, Technoclone) with 1:4,000 final dilution of Dade Innovin (Siemens) to activate coagulation through the extrinsic pathway. Thrombin generation was measured through the cleavage of a fluorogenic substrate (Z-Gly-Gly-Arg-AMC). Fluorescence units were converted to thrombin concentration by calibrating fluorescence units against known concentrations of thrombin, using the Technothrombin calibration kit (Technoclone). Thrombomodulin (TM) was found to reduce thrombin generation in the thrombin generation assay (TGA) in fVIII-deficient plasma (HA).

From this experiment, 1.25 nM and 5 nM TM were selected for subsequent experiments. The high TM concentration used was lower than for NP, mainly because the total thrombin generation in HA plasma in the assay conditions used was lower.

The effects of both FL α₁AT Pitts C232S and FL α₁AT Pitts C232S P2KP1'K on HA and HB plasma were comparable to the results from pooled NP. FL α₁AT Pitts inhibited thrombin generation in the presence and absence of TM in both HA (fVIII-deficient) and HB plasma (fIX-deficient) (Figure 8). FL α₁AT Pitts C232S P2KP1'K could rescue the effect of TM on thrombin generation in both fVIII- and fIX-deficient plasma and had no effect in the absence of TM (Figures 9 and 10). This indicates that FL α₁AT Pitts C232S P2KP1'K can inhibit APC and have a procoagulant effect in factor-deficient plasma. This means it could potentially promote clot formation and reduce bleeding in hemophilia patients. The magnitude of this procoagulant effect will be determined by the relative contribution of the protein C system to the reduction in thrombin generation *in vivo.* The *in vitro* experiments shown here cannot be used to predict the likely efficacy of this mutant *in vivo,* however they do show that in complex plasma systems FL α₁AT Pitts C232S P2KP1'K can inhibit APC and does not interfere with the procoagulant pathways, and that these effects are independent of the presence or absence of fIX and fVIII.

In order to verify our *in vitro* data we wanted to use *in vivo* mouse models of hemophilia. However, to verify that the effect of human α₁AT on mouse plasma would be comparable to the effect seen in human plasma we first performed a TGA in mouse plasma. This was done using a modified TGA protocol (Bunce *et al,* 2011; Ivanciu *et al,* 2011). These modifications were necessary because of the increased concentrations of inhibitory proteins in mouse plasma that preclude TGA assays under standard conditions (Tchaikovski et al, 2007; Bunce *et al,* 2011; Ivanciu *et al,* 2011). Comparable to the human system, there was no thrombin generation in HB mouse plasma under the baseline conditions of the assay. Therefore, we performed a titration spiking in different concentrations of Innovin. A concentration of 1:12,000 Innovin was chosen for subsequent assays.

Because no murine TM was available, we used soluble human TM as used in the human plasma TGAs to promote APC formation in the mouse TGA assay. The concentration required to see any effect of human TM in HB mouse plasma was ∼ 100-fold higher than seen in human plasma. This could be explained by the observation that human TM knock-in mice show reduced ability to activate murine PC (Raife *et al,* 2011). This indicated that human TM is less potent at promoting murine PC activation than murine TM. A concentration of 750nM human TM was used in subsequent experiments.

Different concentrations of both FL α₁AT Pitts C232S and FL α₁AT Pitts C232S P2KP1'K were then added to the determined conditions in mouse HB plasma both in the absence and presence of TM to compare the effect of these mutants in mouse plasma to the previous TGA results in human plasma.

FL α₁AT Pitts C232S reduced thrombin generation in mouse HB plasma in the absence of TM as seen in human plasma (Figure 11A). However in the presence of TM, at the lowest α₁AT concentration, there was a partial rescue of thrombin generation (Figure 11B). This may potentially be due to a difference in the relative rates of inhibition for murine thrombin and murine APC by FL α₁AT Pitts compared to the rates seen in humans, such that the generated APC is inhibited prior to thrombin inhibition. When the concentration of FL α₁AT Pitts C232S is increased to such levels that all APC has been inhibited, thrombin is also inhibited. This could explain the results seen in Figure 11B, but was not further investigated. FL α₁AT Pitts C232S P2KP1'K rescued thrombin generation knocked-down by TM addition in HB mouse plasma as it did in human plasma (Figure 11D). However, when FL α₁AT Pitts C232S P2KP1'K was added to HB mouse plasma in the absence of TM, an increase in thrombin generation was also observed (Figure 11C). It is possible that this effect relates to the method of blood collection employed for these experiments. To collect plasma, the tail was transected and blood collected into citrate. This was then spun down and the plasma removed and frozen. The injury inflicted for blood collection leads to activation of the coagulation system and may cause generation of APC in the plasma prior to the experiment. Additionally, mice do not have PCI in their plasma (Zechmeister-Machhart et al, 1996), which may increase the circulating half-life of APC, such that it is not inactivated prior to the TGA assay. An alternative explanation, which cannot be ruled out at present would be an off-target procoagulant effect in mouse plasma. However, since this effect is not seen in human plasma, this would involve the inhibition of a mouse-specific anticoagulant protease.

To investigate a potential *in vivo* effect of FL α₁AT Pitts C232S P2KP1'K and to determine if it could potentially be useful as either a prophylactic agent or a treatment for hemophilia we used two *in vivo* mouse assays; tail clip and a cremaster arteriole laser injury model. The mice used were male, fIX knockout mice on the BALB/c background (Lin *et al,* 1997; Ivanciu *et al,* 2011).

In the tail clip assay, protein or buffer was injected through the tail vein and after a 5 min incubation period, the tail was transected at a diameter of 3 mm and placed in 14 ml 37°C saline solution in a 37°C water bath. Blood was collected for 10 min and the resulting blood loss was quantified by measuring total hemoglobin after red cell lysis by measuring absorbance at 575 nm (Ivanciu *et al,* 2011). The volume blood loss was calculated by making a standard curve where known volumes of blood collected by tail transection were processed in a similar manner to the tail clip samples. After red cell lysis, the absorbance at 575 nm was determined and plotted against the volume blood loss to generate the standard curve. Tail clip assays showed a potent procoagulant effect of FL α₁AT Pitts C232S P2KP1'K (Figure 12). At the dose of 15 mg/kg, blood loss of the HB mice was restored to the level of WT mice injected with PBS (Figure 12). Lower dose FL α₁AT Pitts C232S P2KP1'K also showed a trend to reduction of bleeding with respect to HB mice although this was not statistically significant. FL α₁AT Pitts C232S showed no significant effect on blood loss at 7.5 mg/kg.

Another *in vivo* model used for evaluating procoagulant agents is the intravital cremaster arteriole laser-induced injury model (Falati et al, 2002). In this system, a cannula is inserted into the mouse jugular vein to allow infusion of the therapeutic protein as well as fluorescently-tagged antibodies to fibrin and platelets. The cremaster muscle is then spread out for imaging. Clot formation after laser-induced injury to the arterioles is imaged and quantified using fluorescence microscopy.

For an overall qualitative assessment, injuries were sorted into three categories: no clot (no fluorescence detected), platelet clot (only platelets visible, these clots were generally unstable and dissolved over the course of the imaging) and platelets + fibrin (both platelet and fibrin fluorescence visible and clot remained stable over the course of the imaging). This showed that there was a dose-dependent increase in stable platelet and fibrin clot formation with increasing concentration of FL α₁AT Pitts C232S P2KP1'K (Figure 13). All images were quantified for platelet and fibrin fluorescence. The median value for each timepoint was calculated and the results plotted in Figure 14. These data included the quantification from all images, regardless of their category assigned for Figure 13. The plots of the median show that control or FL α₁AT Pitts C232S infused mice exhibit no clot formation, whereas both high and low dose FL α₁AT Pitts C232S P2KP1'K showed platelet aggregation and fibrin deposition at the site of the injury. No difference could be detected between the two doses in terms of platelet aggregation. For fibrin, there was a dose-dependent increase in fibrin deposition for FL α₁AT Pitts C232S P2KP1'K and no fibrin for either control or FL α₁AT Pitts C232S infused mice (Figure 14).

Taken together, these results show that FL α₁AT Pitts C232S P2KP1'K has a procoagulant effect in both *in vitro* assays and *in vivo* models of hemophilia. The *in vivo* experiments were all done in mouse models of hemophilia B, however TGA results in human plasma (Figures 9 and 10) and the proposed mechanism of action of FL α₁AT Pitts C232S P2KP1'K indicate that its procoagulant effect should be independent of fIX or fVIII deficiency and could therefore be used in both hemophilia A and B. The procoagulant effect seen *in vivo* was sufficient to reduce bleeding to the same levels as seen for WT mice (Figure 12) indicating that serpins that inhibit APC can be used for treatment of bleeding disorders and not only as a prophylactic or an adjuvant to existing treatments.

A targeted random mutagenesis strategy was also employed on the α₁AT scaffold in order to explore potential additional APC specific mutants on the α₁AT scaffold.

Three different α₁AT variant libraries were generated on the FL α₁AT Pitts C232S background: one randomised at P2, one randomised at P1' and a third library randomised at both P2 and P1'. The resulting plasmid libraries were transformed into the Rosetta2(DE3)pLysS expression strain and protein expressed in 96-well plates. Bacteria were lysed and the lysates assayed for thrombin and APC inhibition.

For the single variant libraries, 88 colonies were assayed per library. For the double (P2P1') variant library, 460 colonies were assayed. FL α₁AT Pitts and FL α₁AT Pitts P2KP1'K (the lead APC specific variants) were expressed on all assay plates as a reference. Variants with higher or equal APC inhibitory activity (lower or equal residual APC activity) and lower or equal thrombin inhibitory activity (higher or equal residual thrombin activity) compared to P2KP1'K α₁AT were deemed candidates for variants specific for APC over thrombin. A subset of candidate variants was picked and re-assayed in the same setup to verify the results from the first screen. Mutants showing similar properties in both assays were then sequenced. The resulting sequences are shown in Table 9. To verify the ability of this assay to pick out variants, which were specific for APC over thrombin, nine variants identified in Table 9 were expressed on a larger scale in *E. coli* and purified. The second-order rate constant of inhibition with respect to thrombin and APC was then determined for each mutant. The results are shown in Table 10. These results confirmed that all variants tested had a higher specificity for APC than for thrombin unlike the FL α₁AT Pitts C232S variant.

Certain types of residues were favored in the α₁AT Pitts scaffold at both P2 and P1' positions (Table 9). Specificity was primarily conferred by the presence of bulky polar (Q, N, Y) bulky hydrophobic (W) or positively charged residues (R, H, K) at P2 and P1'. Other residues seen at these positions included C, A, T, S and V. These medium to small residues were accompanied in the double variant library by a complementing large positively charged residue (R, K) or large polar residue (Y, N, Q) at the other position, which likely has a larger influence on the specificity swap. However, there may be a cooperative effect of these mutations as well, especially where the P1' is R. P1'R showed variable results in the single variant library screen and may have some residual thrombin inhibitory activity. The P2 P is known to be important for thrombin cleavage of substrates (Gallwitz *et al,* 2012). Simple removal of this residue coupled to a specificity swapping mutation at P1' may be sufficient to generate an APC specific inhibitor with little residual thrombin inhibitory activity. Especially T at P2 might have some effect on its own as, out of its partnering residues at P1' (Q, N, Y, R), only R was identified in the single residue P1' variant library as being sufficient to cause a specificity swap by itself.

Interestingly, non-specific mutants that clustered around the α₁AT Pitts control were shown to be largely α₁AT Pitts. All retained the P2 P, showing its importance in maintaining thrombin inhibitory activity. The P1' was more variable, consistent with the distribution of mutants in the P1' variant library. Comparing the spread of variants in the P2 and P1' libraries, thrombin seemed to be more tolerant to P1' than to P2 mutations. However, the appearance of favorable residues in the double variant libraries that were not present in the single variant libraries indicate that the effects of these residues on specificity may be cooperative and double mutations may be more effective than single mutations at increasing specificity.

The random mutagenesis results presented above showed that it was possible to generate mutants that showed specificity for APC over thrombin other than the lysine mutants already identified. So far, the random mutagenesis strategy was used, assuming that once specificity over thrombin was obtained, these mutants would also show some degree of specificity for APC over other procoagulant proteases. To test this, the PT and aPTTs of the random mutants in Table 10 were tested. These results are displayed in Table 11. None of the mutants had a significant effect on the PT, unlike previously shown for FL α1AT Pitts C232S (Figure 4). This provides indication, that the mutants have largely lost their inhibitory activity towards procoagulant proteases. However, aPTTs are more sensitive to the presence of inhibitors. Therefore, the measure of the aPTT might give a more accurate representation of smaller residual inhibition. Previous experiments (Figure 5) showed that FL α1AT Pitts C232S rendered plasma unclottable in the aPTT assay. In contrast, only one of the mutants evaluated here showed this effect (P2TP1'N). Some mutants, such as P2KP1'H and P2KP1'N showed only a relatively small prolongation of the aPTT and were therefore potentially interesting.

From these results, we selected four mutants, P2R, P2QP1'K, P2KP1'H and P2KP1'N (all on the Pitts, P1R background). These showed either a high inhibition of APC over thrombin (Table 10) and for some also a low prolongation of the aPTT (P2KP1'H and P2KP1'N). Because they did not prolong the PT, it is unlikely that they inhibit TF:fVIIa. The most likely candidates for prolongation of the aPTT would be inhibition of fXIa or fXa. Of these, fXa inhibition would most inhibit the initial stages of coagulation and was therefore considered to be a more significant barrier for a successful inhibitor. Inhibition constants for the inhibition of fXa by these four mutants were therefore determined as described above (Table 12). P2R showed significant inhibition of fXa, which may be the reason for its prolongation of the aPTT. The other three mutants showed lower fXa inhibition, but none of the mutants were as specific as the previously identified P2KP1'K mutant. Nevertheless, P2KP1'N, P2QP1'K and P2KP1'H represent additional promising candidates for further development as they show specificity for APC over thrombin and fXa. In addition, their inhibition of APC is roughly 2-fold increased with respect to the P2KP1'K mutant described earlier. This faster inhibition might in part compensate for the slightly reduced specificity.

However, these results indicated that selecting for specificity for APC over thrombin is not completely sufficient to design an inhibitor which also shows specificity over fXa and other procoagulant proteases. Therefore, the random mutagenesis strategy was extended further. Mutants, which previously had been selected for specificity for APC over thrombin, were rescreened against fXa and mutants selected, which had reduced fXa inhibition, while maintaining low thrombin inhibition and APC inhibition.

Four additional mutants were identified from this additional screen. These all had the P1R mutation and in addition had either P2RP1'A, P2RP1'Q, P2WP1'I or P2WP1'H. To verify the specificity of these mutants, an initial experiment was performed, using only one concentration of serpin and testing its inhibition of thrombin and fXa. APC inhibition was not considered at this stage, because mutants were selected based on their low inhibition of thrombin and fXa. Serpin and protease were incubated for different times and at the indicated timepoints, the reaction was stopped by addition of an excess of chromogenic substrate. Residual protease activity was divided by the initial protease activity and the natural log of this value plotted against time (Figure 15). The slope of this line divided by the serpin concentration gives an estimate of the second-order rate constant of inhibition. These assays showed that while all mutants hardly inhibited thrombin, with the fastest inhibitor, P2WP1'H having a second-order rate constant of ∼50.3 M⁻¹.s⁻¹ (compare to the inhibition constant of FL α₁AT Pitts C232S, 2.928 x 10⁵ M⁻¹.s⁻¹ ). However, P2RP1'A, P2WP1'I and P2WP1'H showed significant inhibition of fXa. The second-order rate constant was only ∼10-fold reduced for these mutants compared to FL α₁AT Pitts C232S (4,070.1 M⁻¹.s⁻¹ for P2RP1'A compared to 4.13 x 10⁴ M⁻¹.S⁻¹ for FL α₁AT Pitts C232S). P2RP1'A, P2WP1'I and P2WP1'H showed similar fXa inhibition to each other.

Only one mutant showed significant selectivity against both thrombin and fXa. This mutant had a P2RP1'Q in addition to the Pitts (P1R) mutation. Because of its selectivity, it was interesting for more thorough investigation. Previous results from both the random and rational mutagenesis studies indicated that R and K residues perform reasonably similarly. We therefore also generated a P2KP1'Q mutant on the P1R background as it was expected from the results shown here to have similar properties. The results from measurements of inhibition constants and aPTTs (experiments performed as described before) for both mutants are shown in Table 13. The P2KP1'K mutant is shown for comparison. Both P2KP1'Q and P2RP1'Q showed very low inhibition of thrombin and fXa. In addition, there was also hardly any effect on the aPTT. APC inhibition was significant, being only slightly reduced in comparison to P2KP1'K. Therefore, these two mutants would be expected to perform similarly to P2KP1'K and may represent other potentially promising alternative molecules for further development.

We evaluated inhibition of murine thrombin and APC by FL α₁AT Pitts C232S P2KP1'K. Thrombin and APC were obtained from recombinant sources. The proteases used are truncated with respect to the plasma version, including only the EGF2-protease domains for APC (Gla-domainless APC) and the protease domain for thrombin. Therefore, we also tested the human versions of these proteases to ensure that any differences were due to a species difference, rather than a construct difference. Human and murine thrombin showed none or very little reactivity with FL α₁AT Pitts C232S P2KP1'K by SDS-PAGE, indicating that in this respect, results from model systems would be relevant to the human system. Second-order rate constants of inhibition were (8.14 ± 0.58) x 10³ M⁻¹.s⁻¹, for human Gla-domainless APC, (3.80 ± 0.37) x 10³ M⁻¹.s⁻¹ for murine Gla-domainless APC, compared to (14.88 ± 1.87) x 10³ M⁻¹.s⁻¹ for human plasma APC. These results provided indication that while the reactivity of the mutant in mouse models would likely be lower than in humans, i.e. the relative dose for the same effect might need to be higher, the effect in terms of protease inhibition is likely to be similar.

The data presented show as a proof-of-principle that the serpin scaffold can be used to generate specific APC inhibitors, using only very few mutations, that these inhibitors can have procoagulant activities both *in vitro* and *in vivo* and as such show promise as procoagulant agents for treatment and prophylaxis of bleeding disorders such as hemophilia.

### References

Berntorp E (2009) Haemophilia 15: 3-10
Bertina RM, et al (1994) Nature 369: 64-67
Bohn RL et al (2004) Haemophilia 10: 63-68
Bolton-Maggs PHB & Pasi KJ (2003) Lancet 361: 1801-1809
Brettler DB (1996) Baillieres Clin. Haematol.9: 319-329
Brummel-Ziedins KE et al (2011) J. Thromb. Haemost. 9: 2262-2267
Bunce MW et al (2011). Blood 117: 290-298
Butenas S et al (2006) J. Thromb. Haemost. 4: 2411-2416
Carrell R et al (1985) Trends in Biochemical Sciences 10: 20-24
Chuansumrit A et al (2000) Haemophilia 6: 61-65
De Nanteuil G et al (2006) J. Med. Chem. 49: 5047-5050
Di Minno MND et al (2010) Haemophilia 16: e190-201
DiMichele D (2007) J. Haematol. 138: 305-315
Duckers C et al (2010) Blood 115: 879-886
Elisen MGLM et al (1998) Blood 91: 1542-1547
Escobar MA (2010) Haemophilia 16 Suppl 3: 29-34
Escobar M & Sallah S et al (2013) J. Thromb. Haemost. 11, 1449-1453.
Falati S et al (2002) Nat. Med. 8: 1175-1181
Fortenberry YM et al (2011) J. Thromb. Haemost. 9: 861-863
Franchini M & Lippi G (2010) Thrombosis Research 125: 119-123
Fukudome K et al (1996) J. Biol. Chem. 271: 17491-17498
Gallwitz et al (2012) PLoS ONE 7(2): e31756
Gettins PGW (2002) Chem. Rev. 102: 4751-4803
Gringeri A et al (2003) Blood 102: 2358-2363
Haya S et al (2007) Haemophilia 13 Suppl 5: 52-60
Heeb MJ & Griffin JH (1988) J. Biol. Chem. 263: 11613-11616
Heeb MJ et al (1990) J. Biol. Chem. 265: 2365-2369
Hopkins et al (1993) Biochemistry 32; 7650-7657
Hua B et al (2009) Haematologica 94: 881-884
Huntington JA et al (2000) Nature 407: 923-926
Irving et al (2000) Genome Res. 10; 1845-1864
Ivanciu L et al (2011) Nat. Biotechnol. 29: 1028-1033
Kalsheker N (1989) Biosci. Rep. 9: 129-138
Laurell CB et al (1977) Clin Sci Mol Med 52: 457-461
Laurell M et al (1990) Blood 76: 2290-2295
Lee M et al (2006) Haemophilia 12 Suppl 3: 1-7
Li W & Huntington JA (2008) J. Biol. Chem. 283: 36039-36045
Li W et al (2008) Proc. Natl. Acad. Sci. U.S.A. 105: 4661-4666
Lin HF et al 1997) Blood 90: 3962-3966
Lowe, G.D. & Ludlam, C.A. (2008) J. Thromb. Haemost.6, 1982-1983.
Lu D et al (1996) Blood 87: 4708-4717
Mannucci PM (2003) J. Thromb. Haemost. 1: 1349-1355
Mannucci PM (2008) Haemophilia 14 Suppl 3: 10-18
Mather T et al (1996) EMBO J 15: 6822-6831
Meijers JC et al 1988) Biochemistry 27: 4231-4237
Mosnier LO et al (2001) Thromb. Haemost. 86: 1057-1064
Nagel K. et al (2011) Haemophilia, 17, 872-874.
Negrier C et al2006) Haemophilia 12 Suppl 6: 48-52- discussion 52-3
Owen MC et al (1983) N. Engl. J. Med. 309: 694-698
Pratt CW & Church FC (1992) J. Biol. Chem. 267: 8789-8794
Pratt CW et al (1992) J. Biol. Chem. 267: 8795-8801
Raife TJ et al (2011) Arterioscler. Thromb. Vase. Biol. 31: 2509-2517
Schechter I et al (1967)Biochem Biophys Res Comm 27: 157-162
Stearns-Kurosawa DJ et al (1996) PNAS. U.S.A. 93: 10212-10216
Suzuki K et al (1983) J. Biol. Chem. 258: 163-168
Suzuki K et al (1984) Journal of Biochemistry 95: 187-195
Tagariello, G et al (2009) Blood, 114, 779-784.
Tchaikovski SN et al (2007) J. Thromb. Haemost. 5: 2079-2086
Teitel JM (1999) Haemophilia 5 Suppl 3: 43-49
Teitel, JM & Sholzberg, M (2013) Blood Reviews 27 103-109
Turecek PL, et al (2004) Haemophilia 10 Suppl 2: 3-9
Ullman M et al(2006) Haemophilia 12 Suppl 6: 74-9; discussion 79-80
World Federation of Hemophilia (2011) 2010 WFH Global Survey Report
Yamasaki M et al (2011) EMBO Rep. 12: 1011-1017
Zechmeister-Machhart M et al (1996) Immunopharmacology 32: 96-98

**Table 1**

| **Second-order rate constants of inhibition (mM⁻¹.s⁻¹)** | | | |
|---|---|---|---|
| **Variant** | **Thrombin** | **APC** | **Fold inhibition APC/thrombin** |
| A22 WT PCI | 28.21 ± 1.51 | 0.68 ± 0.032 | 0.02 |
| A22 P1'K PCI | 0.022 ± 0.0024 | 0.88 ± 0.074 | 40 |
| A22 P2KP1'K PCI | ∼ 0.03* | 0.28 ± 0.013 | 9.3 |
| FL α₁AT Pitts C232S | 292.76 ± 17.60 | 108.16 ± 7.086 | 0.4 |
| FL α₁AT Pitts C232S P2K | 0.051 ± 0.0028 | 64.82 ± 7.14 | 1,271 |
| FL α₁AT Pitts C232S P1'K | 0.17 ± 0.017 | 95.66 ± 13.70 | 563 |
| FL α₁AT Pitts C232S P2KP1'K | no inhibition after 4 h | 15.14 ± 1.68 | no thrombin inhibition |

**Table 2**

| **Second-order rate constants of inhibition (mM⁻¹.s⁻¹)** | | | |
|---|---|---|---|
| **Variant** | **Thrombin + heparin** | **APC + heparin** | **Fold inhibition APC/Thrombin** |
| A22 WT PCI | 1310.32 ± 218.72 | 564.47 ± 71.29 | 0.4 |
| A22 P1'K PCI | 0.017 ± 0.0019 | 321.54 ± 31.94 | 18914 |
| A22 P2KP1'K PCI | 0.11 ± 0.040* | 146.38 ± 18.85 | 1331.7 |

**Table 3**

| **Second-order rate constants of inhibition (mM⁻¹.s⁻¹)** | | |
|---|---|---|
| **Variant** | **fXa** | **Fold inhibition APC/fXa** |
| A22 WT PCI | 10.31 ± 0.73 | 0.07 |
| A22 P1'K PCI | 0.52 ± 0.079 | 1.7 |
| A22 P2KP1'K PCI | no detectable inhibition | no fXa inhibition |
| FL α₁AT Pitts C232S | 41.33 ± 2.36 | 2.6 |
| FL α₁AT Pitts C232S P2K | 3.93 ± 0.31 | 16.5 |
| FL α₁AT Pitts C232S P1'K | 4.89 ± 0.16 | 19.6 |
| FL α₁AT Pitts C232S P2KP1'K | 0.12 ± 0.010 | 126.2 |

**Table 4**

| **Second-order rate constants of inhibition (mM⁻¹.s⁻¹)** | | | |
|---|---|---|---|
| **Variant** | **fXIa** | **APC** | **Inhibition of APC/fXIa** |
| A22 WT PCI | 8.59 ± 0.43 | 0.68 ± 0.032 | 0.08 |
| A22 P2KP1'K PCI | 0.023 ± 0.0052 | 0.28 ± 0.013 | 12.2 |

**Table 5**

| **Second-order rate constants of inhibition (mM⁻¹.s⁻¹)** | | | |
|---|---|---|---|
| **Variant** | **Thrombin** | **APC** | **Inhibition of APC/Thrombin** |
| A22 WT PCI | 28.21 ± 1.51 | 0.68 ± 0.032 | 0.02 |
| A22 P2KP1'K PCI | ∼ 0.03* | 0.28 ± 0.013 | 9.3 |
| A22 D8 PCI (P4QP2RP1'N) | 0.084 ± 0.0016 | 1.00 ± 0.15 | 11.9 |
| A22 4.H11 PCI (P4KP2RP1'H) | 0.021 ± 0.0012 | 0.45 ± 0.15 | 21.4 |
| A22 2.B10 PCI (P4SP2LP1'K) | 0.016 ± 0.00090 | 0.43 ± 0.0086 | 26.9 |
| A22 5.E12 PCI (P4HP2RP1'V) | 0.023 ± 0.0021 | 0.26 ± 0.035 | 11.3 |

**Table 6**

| **Second-order rate constants of inhibition (mM⁻¹.s⁻¹)** | | | |
|---|---|---|---|
| **Variant** | **fXIa** | **APC** | **Inhibition of APC/fXIa** |
| FL α₁AT Pitts C232S | 398.88 ± 13.012 | 108.16 ± 7.086 | 0.3 |
| FL α₁AT Pitts C232S P2KP1'K | 0.47 ± 0.037 | 15.14 ± 1.68 | 32.2 |

**Table 7**

| | P6 | P5 | P4 | P3 | P2 | P1 | P1' | P2' | P3' | P4' |
|---|---|---|---|---|---|---|---|---|---|---|
| WT | T | I | **F** | T | **F** | R | **S** | A | R | L |
| P2KP1'K | T | I | **F** | T | **K** | R | **K** | A | R | L |
| E7 | T | I | **S** | T | **H** | R | **R** | A | R | L |
| E10 | T | I | **R** | T | **Q** | R | **V** | A | R | L |
| E11 | T | I | **T** | T | **L** | R | **Y** | A | R | L |
| D8 | T | I | **Q** | T | **R** | R | **N** | A | R | L |
| H11 | T | I | **A** | T | **Q** | R | **Y** | A | R | L |

**Table 8**

| | P6 | P5 | P4 | P3 | P2 | P1 | P1' | P2' | P3' | P4' |
|---|---|---|---|---|---|---|---|---|---|---|
| WT | T | I | **F** | T | **F** | R | **S** | A | R | L |
| P2KP1'K | T | I | **F** | T | **K** | R | **K** | A | R | L |
| 2.B10 | T | I | **S** | T | **L** | R | **K** | A | R | L |
| 3.B11 | T | I | **F** | T | **F** | R | **R** | A | R | L |
| 3.C3 | T | I | **V** | T | **R** | R | **I** | A | R | L |
| 3.E8 | T | I | **F** | T | **R** | R | **K** | A | R | L |
| 3.G10 | T | I | **C** | T | **L** | R | **K** | A | R | L |
| 3.G11 | T | I | **W** | T | **W** | R | **N** | A | R | L |
| 3.H11 | T | I | **W** | T | **W** | R | **N** | A | R | L |
| 4.E4 | T | I | **K** | T | **D** | R | **M** | A | R | L |
| 4.F7 | T | I | **F** | T | **V** | R | **K** | A | R | L |
| 4.F10 | T | I | **R** | T | **R** | R | **I** | A | R | L |
| 4.H7 | T | I | **G** | T | **I** | R | **R** | A | R | L |
| 4.H11 | T | I | **K** | T | **R** | R | **H** | A | R | L |
| 5.D2 | T | I | **T** | T | **R** | R | **V** | A | R | L |
| 5.E7 | T | I | **L** | T | **R** | R | **I** | A | R | L |
| 5.E12 | T | I | **H** | T | **R** | R | **V** | A | R | L |

**Table 9**

| | P6 | P5 | P4 | P3 | P2 | P1 | P1' | P2' | P3' | P4' |
|---|---|---|---|---|---|---|---|---|---|---|
| α1AT WT | L | E | A | I | **P** | M | **S** | I | P | p |
| α1AT Pitts | L | E | A | I | **P** | R | **S** | I | P | P |
| P2.G11 | L | E | A | I | **K** | R | **S** | I | P | P |
| P2.F10 | L | E | A | I | **R** | R | **S** | I | P | P |
| P2. D8 | L | E | A | I | **K** | R | **S** | I | P | P |
| P2.G8 | L | E | A | I | **R** | R | **S** | I | P | P |
| P2.E7 | L | E | A | I | **R** | R | **S** | I | P | p |
| P2.D10 | L | E | A | I | **R** | R | **S** | I | P | P |
| P2.G4 | L | E | A | I | **R** | R | **S** | I | p | p |
| P2. F4 | L | E | A | I | **R** | R | **S** | I | P | P |
| P1'.H8 | L | E | A | I | **P** | R | **E** | I | p | P |
| P1'.A11 | L | E | A | I | **P** | R | **R** | I | p | P |
| P1'.F10 | L | E | A | I | **P** | R | **E** | I | p | P |
| P1.F9 | L | E | A | I | **P** | R | **K** | I | p | p |
| P1'.F4 | L | E | A | I | **P** | R | **E** | I | P | P |
| 4.G9 | L | E | A | I | **T** | R | **N** | I | P | P |
| 4.G4 | L | E | A | I | **Q** | R | **K** | I | p | p |
| 3.E5 | L | E | A | I | **R** | R | **A** | I | P | p |
| 3.B6 | L | E | A | I | **S** | R | **R** | I | p | p |
| 3.B2 | L | E | A | I | **K** | R | **N** | I | P | p |
| 3.A10 | L | E | A | I | **T** | R | **Y** | I | P | P |
| 2.H1 | L | E | A | I | **R** | R | **H** | I | P | P |
| 2.C6 | L | E | A | I | **T** | R | **R** | I | P | P |
| 1.H10 | L | E | A | I | **V** | R | **R** | I | P | P |
| 1.B11 | L | E | A | I | **R** | R | **C** | I | P | P |
| 1.A12 | L | E | A | I | **K** | R | **H** | I | P | P |
| 2.E5 | L | E | A | I | **T** | R | **R** | I | P | P |
| 3.G9 | L | E | A | I | **Y** | R | **R** | I | p | p |
| 3.F4 | L | E | A | I | **A** | R | **R** | I | P | P |
| 3.C9 | L | E | A | I | **C** | R | **K** | I | P | P |
| 2.H5 | L | E | A | I | **K** | R | **N** | I | P | P |
| 2.E7 | L | E | A | I | **W** | R | **N** | I | P | P |
| 1.B2 | L | E | A | I | **S** | R | **R** | I | P | P |
| 5.C12 | L | E | A | I | **H** | R | **N** | I | P | P |
| 5.A6 | L | E | A | I | **R** | R | **N** | I | P | P |
| 4.E1 | L | E | A | I | **P** | R | **K** | I | P | P |
| 4.C12 | L | E | A | I | **N** | R | **N** | I | P | P |
| 3.F8 | L | E | A | I | **T** | R | **M** | I | P | P |
| 3.C10 | L | E | A | I | **T** | R | **H** | I | P | P |
| 2.E8 | L | E | A | I | **K** | R | **S** | I | P | P |
| 1.H9 | L | E | A | I | **T** | R | **Q** | I | P | P |

**Table 10**

| **Second-order rate constants of inhibition (mM⁻¹.s⁻¹)** | | | |
|---|---|---|---|
| **Variant** | **Thrombin** | **APC** | **Inhibition of APC/thrombin** |
| FL α₁AT Pitts C232S | 292.76 ± 17.60 | 108.16 ± 7.086 | 0.4 |
| FL α₁AT Pitts C232S P2KP1'K | no inhibition after 4 h | 15.14 ± 1.68 | No thrombin inhibition |
| FL α₁AT Pitts C232S P2R | 0.042 ± 0.0024 | 61.12 ± 6.26 | 1455.2 |
| FL α₁AT Pitts C232S P1'R | 0.68 ± 0.068 | 131.57 ± 13.32 | 193.5 |
| FL α₁AT Pitts C232S P1'E | 0.15 ± 0.015 | 2.99 ± 0.29 | 19.9 |
| FL α₁AT Pitts C232S P2TP1'N | 0.27 ± 0.047 | 62.37 ± 2.46 | 231.0 |
| FL α₁AT Pitts C232S P2TP1'Y | 0.023 ± 0.0014 | 5.70 ± 0.83 | 247.8 |
| FL α₁AT Pitts C232S P2QP1'K | 0.0038 ± 0.0013 | 33.41 ± 6.36 | 8792.1 |
| FL α₁AT Pitts C232S P2KP1'H | no inhibition after 2 h | 28.84 ± 3.05 | No thrombin inhibition |
| FL α₁AT Pitts C232S P2KP1'N | 0.015 ± 0.0026 | 37.80 ± 2.48 | 2520.0 |
| FL α₁AT Pitts C232S P2RP1'C | 0.034 ± 0.0094 | 24.55 ± 2.15 | 722.1 |

**Table 11**

| **Variant** | **PT (s)** | **aPTT (s)** |
|---|---|---|
| Plasma | 27.2 ± 0.8 | 60.3 |
| FL α₁AT Pitts C232S P2K | 27.0 ± 0.5 | 107.2 |
| FL α₁AT Pitts C232S P1'K | 27.1 ± 0.4 | 228.1 |
| FL α₁AT Pitts C232S P2R | 27.8 ± 0.4 | 111.6 |
| FL α₁AT Pitts C232S P1'R | 28.1 ± 0.5 | 287 |
| FL α₁AT Pitts C232S P1'E | 27.2 ± 0.4 | 84 |
| FL α₁AT Pitts C232S P2TP1'N | 29.5 ± 0.9 | >300 |
| FL α₁AT Pitts C232S P2TP1'Y | 28.3 ± 0.8 | 185.4 |
| FL α₁AT Pitts C232S P2QP1'K | 27.9 ± 0.3 | 111.5 |
| FL α₁AT Pitts C232S P2KP1'H | 27.4 ± 0.9 | 77.8 |
| FL α₁AT Pitts C232S P2KP1'N | 27.8 ± 0.3 | 81.9 |
| FL α₁AT Pitts C232S P2RP1'C | 28.5 ± 0.6 | ND |

**Table 12**

| **Second-order rate constants of inhibition (mM⁻¹.s⁻¹)** | | |
|---|---|---|
| **Variant** | **fXa** | **Inhibition of APC/fXa** |
| FL α₁AT Pitts C232S | 41.33 ± 2.36 | 2.6 |
| FL α₁AT Pitts C232S P2KP1'K | 0.12 ± 0.010 | 126.2 |
| FL α₁AT Pitts C232S P2R | 4.79 ± 0.58 | 12.8 |
| FL α₁AT Pitts C232S P2QP1'K | 1.082 ± 0.15 | 30.9 |
| FL α₁AT Pitts C232S P2KP1'H | 0.62 ± 0.040 | 46.5 |
| FL α₁AT Pitts C232S P2KP1'N | 0.91 ± 0.13 | 41.5 |

**Table 13**

| **Second-order rate constants of inhibition (mM⁻¹.s⁻¹)** | | | | **aPTT (s)** |
|---|---|---|---|---|
| **Variant** | **Thrombin** | **APC** | **fXa** | |
| FL α₁AT Pitts C232S | 292.76 ± 17.60 | 108.16 ± 7.086 | 41.33 ± 2.36 | >300 (55.0 ± 3.8) |
| FL α₁AT Pitts C232S P2KP1'K | no inhibition after 4 h | 15.14 ± 1.87 | 0.12 ± 0.010 | 62.1 ± 4.2 (55.0 ± 3.8) |
| FL α₁AT Pitts C232S P2RP1'Q | 0.0054 ± 0.0011 | 8.30 ± 1.11 | 0.13 ± 0.0067 | 55.1 ± 2.8 (49.3 ± 2.5) |
| FL α₁AT Pitts C232S P2KP1'Q | 0.0029 ± 0.0015 | 9.00 ± 0.67 | 0.17 ± 0.010 | 53.9 ± 3.0 (49.3 ± 2.5) |

### Sequences

1 mqlflllclv llspqgaslh rhhpremkkr vedlhvgatv apssrrdftf dlyralasaa
61 psqniffspv sismslamls lgagsstkmq ilegiglnlq kssekelhrg fqqllqelnq
121 prdgfqlslg nalftdlvvd lqdtfvsamk tlyladtfpt nfrdsagamk qindyvakqt
181 kgkivdllkn ldsnavvimv nyiffkakwe tsfnhkgtqe qdfyvtsetv vrvpmmsred
241 qyhylldrnl scrvvgvpyq gnatalfilp segkmqqven glsektlrkw lkmfkkrqle
301 lylpkfsieg syqlekvlps lgisnvftsh adlsgisnhs niqvsemvhk avvevdesgt
361 raaaatgtif tfrsarlnsq rlvfnrpflm fivdnnilfl gkvnrp

SEQ ID NO: 1 Protein C inhibitor (PCI)

Mature protein, including the propeptide, corresponds to residues 20 to 406. Signal sequence corresponds to residues 1-19. Propeptide corresponds to residues 20-25. Residues P4, P2, P1 and P1' of the RCL bold and underlined. 
1 mermlpllal gllaagfcpa vlchpnspld eenltqenqd rgthvdlgla sanvdfafsl
61 ykqlvlkapd knvifsplsi stalaflslg ahnttlteil kglkfnltet seaeihqsfq
121 hllrtlnqss delqlsmgna mfvkeqlsll drftedakrl ygseafatdf qdsaaakkli
181 ndyvkngtrg kitdlikdld sqtmmvlvny iffkakwemp fdpqdthqsr fylskkkwvm
241 vpmmslhhlt ipyfrdeels ctvvelkytg nasalfilpd qdkmeeveam llpetlkrwr
301 dslefreige lylpkfsisr dynlndillq lgieeaftsk adlsgitgar nlavsqvvhk
361 avldvfeegt easaatavki tllsalvetr tivrfnrpfl miivptdtqn iffmskvtnp
421 kqa
 SEQ ID NO: 2 Alpha-1-antichymotrypsin

Mature protein corresponds to residues 26 to 423. Residues P4, P2, P1 and P1' of the RCL bold and underlined 
1 
61 
121 
181 
241 
301 
361 
421 
481 
 SEQ ID NO: 3 C1-esterase inhibitor

Mature protein corresponds to residues 23-500. Residues P4, P2, P1 and P1' of the RCL bold and underlined 
1 mallwgllvl swsclqgpcs vfspvsamep lgrqltsgpn qeqvspltll klgnqepggq
61 talksppgvc srdptpeqth rlarammaft adlfslvaqt stcpnlilsp lsvalalshl
121 algaqnhtlq rlqqvlhags gpclphllsr lcqdlgpgaf rlaarmylqk gfpikedfle
181 qseqlfgakp vsltgkqedd laninqwvke ategkiqefl sglpedtvll llnaihfqgf
241 wrnkfdpslt qrdsfhldeq ftvpvemmqa rtyplrwfll eqpeiqvahf pfknnmsfvv
301 lvpthfewnv sqvlanlswd tlhpplvwer ptkvrlpkly lkhqmdlvat lsqlglqelf
361 qapdlrgise qslvvsgvqh qstlelsevg veaaaatsia msrmslssfs vnrpflffif
421 edttglplfv gsvrnpnpsa prelkeqqds pgnkdflqsl kgfprgdklf gpdlklvppm
481 eedypqfgsp k
 SEQ ID NO: 4 α₂-Antiplasmin

Mature protein corresponds to residues 28-491. Residues P4, P2, P1 and P1' of the RCL for inhibition of chymotrypsin in bold, residues for the inhibition of plasmin underlined. 
1 
61 
121 
181 
241 
301 
361 
421 
 SEQ ID NO: 5 Antithrombin (ATIII)

Mature protein corresponds to residues 33-464. Residues P4, P2, P1 and P1' of the RCL bold and underlined 
1 mkhslnalli fliitsawgg skgpldqlek ggetaqsadp qweqlnnknl smpllpadfh
61 kentvtndwi pegeedddyl dlekifsedd dyidivdsls vsptdsdvsa gnilqlfhgk
121 sriqrlniln akfafnlyrv lkdqvntfdn ifiapvgist amgmislglk getheqvhsi
181 lhfkdfvnas skyeittihn lfrklthrlf rrnfgytlrs vndlyiqkqf pilldfktkv
241 reyyfaeaqi adfsdpafis ktnnhimklt kglikdalen idpatqmmil nciyfkgswv
301 nkfpvemthn hnfrlnerev vkvsmmqtkg nflaandqel dcdilqleyv ggismlivvp
361 hkmsgmktle aqltprvver wqksmtnrtr evllpkfkle knynlveslk lmgirmlfdk
421 ngnmagisdq riaidlfkhq gtitvneegt qattvttvgf mplstqvrft vdrpflfliy
481 ehrtscllfm grvanpsrs
 SEQ ID NO: 6 Heparin cofactor II

Mature protein corresponds to residues 20-499. Residues P4, P2, P1 and P1' of the RCL bold and underlined 
1 mpssvswgil llaglcclvp vslaedpqgd aaqktdtshh dqdhptfnki tpnlaefafs
61 lyrqlahqsn stniffspvs iatafamlsl gtkadthdei leglnfnlte ipeaqihegf
121 qellrtlnqp dsqlqlttgn glflseglkl vdkfledvkk lyhseaftvn fgdteeakkq
181 indyvekgtq gkivdlvkel drdtvfalvn yiffkgkwer pfevkdteee dfhvdqvttv
241 kvpmmkrlgm fniqhckkls swvllmkylg natiifflpd egklqhlene lthdiitkfl
301 enedrrsasl hlpklsitgt ydlksvlgql gitkvfsnga dlsgvteeap lklskavhka
361 vltidekgte aagamflea i pms ippevkf nkpfvflmie qntksplfmg kvvnptqk
 SEQ ID NO: 7 α₁-antitrypsin (α₁AT)

Mature protein corresponds to residues 25-418. Residues P4, P2, P1 and P1' of the RCL bold and underlined 
1 mhlidyllll lvgllalshg qlhvehdges csnsshqqil etgegspslk iapanadfaf
61 rfyyliaset pgkniffspl sisaayamls lgacshsrsq ileglgfnlt elsesdvhrg
121 fqhllhtlnl pghgletrvg salflshnlk flakflndtm avyeaklfht nfydtvgtiq
181 lindhvkket rgkivdlvse lkkdvlmvlv nyiyfkalwe kpfissrttp kdfyvdentt
241 vrvpmmlqdq ehhwylhdry lpcsvlrmdy kgdatvffil pnqgkmreie evltpemlmr
301 wnnllrkrnf ykklelhlpk fsisgsyvld qilprlgftd lfskwadlsg itkqqkleas
361 ksfhkatldv deagteaaaa tsfaikffsa qtnrhilrfn rpflvvifst stqsvlflgk
421 vvdptkp
 SEQ ID NO: 8 Kallistatin

Mature protein corresponds to residues 21-427. Residues P4, P2, P1 and P1' of the RCL bold and underlined 
1 
61 
121 
181 
241 
301 
361 
 SEQ ID NO: 9 Plasminogen activator inhibitor

Mature protein corresponds to residues 24-402. Residues P4, P2, P1 and P1' of the RCL bold and underlined 
1 mkvvpsllls vllaqvwlvp glapspqspe tpapqnqtsr vvqapkeeee deqeaseeka
61 seeekawlma srqqlakets nfgfsllrki smrhdgnmvf spfgmslamt glmlgatgpt
121 etqikrglhl qalkptkpgl lpslfkglre tlsrnlelgl tqgsfafihk dfdvketffn
181 lskryfdtec vpmnfrnasq akrlmnhyin ketrgkipkl fdeinpetkl ilvdyilfkg
241 kwltpfdpvf tevdtfhldk yktikvpmmy gagkfastfd knfrchvlkl pyqgnatmlv
301 vlmekmgdhl aledylttdl vetwlrnmkt rnmevffpkf kldqkyemhe llrqmgirri
361 fspfadlsel satgrnlqvs rvlqrtviev dergteavag ilseitaysm ppvikvdrpf
421 hfmiyeetsg mllflgrvvn ptll
 SEQ ID NO: 10 Protein Z dependent inhibitor

Mature protein corresponds to residues 22-444. Residues P4, P2, P1 and P1' of the RCL bold and underlined 
1 mnwhlplfll asvtlpsics hfnplsleel gsntgiqvfn qivksrphdn ivisphgias
61 vlgmlqlgad grtkkqlamv mrygvngvgk ilkkinkaiv skknkdivtv anavfvknas
121 eievpfvtrn kdvfqcevrn vnfedpasac dsinawvkne trdmidnlls pdlidgvltr
181 lvlvnavyfk glwksrfqpe ntkkrtfvaa dgksyqvpml aqlsvfrcgs tsapndlwyn
241 fielpyhges ismliaplte sstplsaiip histktidsw msimvpkrvq vilpkftava
301 qtdlkeplkv lgitdmfdss kanfakittg senlhvshil qkakievsed gtkasaatta
361 iliarssppw fivdrpflff irhnptgavl fmgqinkp
 SEQ ID NO: 11 Protease nexin 1

Mature protein corresponds to residues residues 20-398 - isoform a. Residues P4, P2, P1 and P1' of the RCL bold and underlined 
1 mpssvswgil llaglcclvp vslaedpqgd aaqktdtshh dqdhptfnki tpnlaefafs
61 lyrqlahqsn stniffspvs iatafamlsl gtkadthdei leglnfnlte ipeaqihegf
121 qellrtlnqp dsqlqlttgn glflseglkl vdkfledvkk lyhseaftvn fgdteeakkq
181 indyvekgtq gkivdlvkel drdtvfalvn yiffkgkwer pfevkdteee dfhvdqvttv
241 kvpmmkrlgm fniqhckkls swvllmkylg nataifflpd egklqhlene lthdiitkfl
301 enedrrsasl hlpklsitgt ydlksvlgql gitkvfsnga dlsgvteeap lklskavhka
361 vltidekgte aagamflea i krk ippevkf nkpfvflmie qntksplfmg kvvnptqk
 SEQ ID NO: 12 Modified Serpin in α₁AT scaffold

Mature protein corresponds to residues 25-418. Residues P4, P2, P1 and P1' of the RCL bold and underlined

## Claims

1. A modified serpin having mutations at residues P1' and P2 and optionally P1 and/or P4 in the reactive center loop (RCL) thereof,
wherein the P1' residue is mutated to Q, H, K or R; and the P2 residue is mutated to H, K or R, and;
wherein said mutations increase the inhibition of one or more anticoagulant proteases selected from activated Protein C and thrombin:thrombomodulin complex relative to the inhibition of one or more procoagulant proteases selected from thrombin, fVIIa, fXa, fIXa and fXIa.

2. A modified serpin according to claim 1 wherein the P1' residue is mutated to K or Q.

3. A modified serpin according to claim 1 or claim 2 wherein the P2 residue is mutated to K.

4. A modified serpin according to claim 1 wherein the P2 and the P1' residues respectively in the modified serpin are KK, RK, RH, KH, RQ or KQ.

5. A modified serpin according to any one of the preceding claims wherein the modified serpin comprises a mutation at P4, optionally wherein the P4 residue is mutated to F, S, R, V, C, W, K, G, L, H, T, Q or A.

6. A modified serpin according to any one of the preceding claims wherein the modified serpin comprises a mutation at P1, optionally wherein the P1 residue is mutated to R.

7. A modified serpin according to any one of claims 1 to 5 wherein the modified serpin has an R residue at the P1 position.

8. A modified serpin according to any one of claims 1 to 7 wherein the mutations in the RCL of the modified serpin consist of mutations at positions P1' and P2; positions P1', P2 and P1; positions P1', P2 and P4; or positions P1', P2, P4 and P1.

9. A modified serpin according to any one of claims 1 to 8 wherein;
the P4 residue in the RCL of the modified serpin is A, the P2 residue is K, and the P1' residue is K;
the P4 residue in the RCL of the modified serpin is A, the P2 residue is R, and the P1' residue is H;
the P4 residue in the RCL of the modified serpin is A, the P2 residue is K, and the P1' residue is H;
the P4 residue in the RCL of the modified serpin is A, the P2 residue is R, and the P1' residue is Q;
the P4 residue in the RCL of the modified serpin is A, the P2 residue is K, and the P1' residue is Q;
the P4 residue in the RCL of the modified serpin is A, the P2 residue is K, the P1 residue is R and the P1' residue is K;
the P4 residue in the RCL of the modified serpin is A, the P2 residue is K, the P1 residue is R and the P1' residue is Q; or
the P4 residue in the RCL of the modified serpin is A, the P2 residue is R, the P1 residue is R and the P1' residue is Q;
the P4 residue in the RCL of the modified serpin is K, the P2 residue is R and the P1' residue is H;
the P4 residue in the RCL of the modified serpin is F, the P2 residue is K and the P1' residue is K;
the P4 residue in the RCL of the modified serpin is F, the P2 residue is R and the P1' residue is K; or
the P4 residue in the RCL of the modified serpin is S, the P2 residue is H and the P1' residue is R.

10. A modified serpin according to any one of the preceding claims that comprises an amino acid sequence that has at least 70% sequence identity to the sequence of a wild-type serpin.

11. A modified serpin according to claim 10 wherein the wild type serpin is selected from the group consisting of α₁-antichymotrypsin (SERPINA3), C1-esterase inhibitor, α₂-antiplasmin (SERPINF2) antithrombin (ATIII) (SERPINC1), heparin cofactor II (HCII) (SERPIND1), protein C inhibitor (PCI) (SERPINA5) or α₁-antitrypsin (α₁AT) (SERPINA1), Kallistatin (SERPINA4), Plasminogen activator inhibitor (SERPINE1), Protease nexin 1 (SERPINE2) and Protein Z-dependent protease inhibitor (SERPINA10).

12. A modified serpin according to any one of claims 1 to 11 comprising an amino acid sequence having at least 70% sequence identity to residues 25-418 of SEQ ID NO: 12, wherein the P4 residue in the RCL of the modified serpin is A, the P2 residue is K, P1 residue is R and the P1' residue is K.

13. A modified serpin according to claim 12 comprising;
(i) the amino acid sequence of residues 25-418 of SEQ ID NO: 12;
(ii) the amino acid sequence of residues 25-418 of SEQ ID NO: 12 with a C to S mutation at residue 256;
(iii) the amino acid sequence of residues 25-418 of SEQ ID NO: 12 with a E to S mutation at residue 25; or
(iv) the amino acid sequence of residues 25-418 of SEQ ID NO: 12 with a E to S mutation at residue 25 and a C to S mutation at residue 256.

14. A nucleic acid encoding a modified serpin according to any one of claims 1 to 13, a vector comprising a nucleic acid encoding a modified serpin according to any one of claims 1 to 13 or a recombinant cell comprising a vector which expresses a modified serpin according to any one of claims 1 to 13.

15. A pharmaceutical composition comprising a modified serpin according to any one of claims 1 to 13 and a pharmaceutically acceptable excipient.

16. A modified serpin according to any one of claims 1 to 13 for use in the treatment of the human or animal body.

17. A modified serpin according to any one of claims 1 to 13 for use in the treatment or prevention of bleeding or the promotion of hemostasis in an individual.

18. A modified serpin for use according to claim 17, wherein the individual has a bleeding disorder, optionally wherein the bleeding disorder is hemophilia.

19. A modified serpin for use according to claim 17 wherein the individual is a trauma patient.

## Patentansprüche

1. Modifiziertes Serpin, das in seiner reaktiven Zentrumsschleife (RCL) Mutationen an den Resten P1' und P2 und gegebenenfalls an P1 und/oder P4 aufweist,
wobei der P1'-Rest zu Q, H, K oder R mutiert ist und der P2-Rest zu H, K oder R mutiert ist und
wobei die Mutationen die Hemmung einer oder mehrerer gerinnungshemmender Proteasen, ausgewählt aus aktiviertem Protein C und Thrombin:Thrombodulin-Komplex, im Vergleich zur Hemmung einer oder mehrerer prokoagulierender Proteasen, ausgewählt aus Thrombin, fVIIa, fXA, fIXa und fXIa, erhöht

2. Modifiziertes Serpin nach Anspruch 1, wobei der P1'-Rest zu K oder Q mutiert ist.

3. Modifiziertes Serpin nach Anspruch 1 oder Anspruch 2, wobei der P2-Rest zu K mutiert ist.

4. Modifiziertes Serpin nach Anspruch 1, wobei der P2- und der P1'-Rest im modifizierten Serpin jeweils KK, RK, RH, KH, RQ oder KQ sind.

5. Modifiziertes Serpin nach einem der vorangegangenen Ansprüche, wobei das modifizierte Serpin eine Mutation an P4 umfasst, wobei der P4-Rest gegebenenfalls zu F, S, R, V, C, W, K, G, L, H, T, Q oder A mutiert ist.

6. Modifiziertes Serpin nach einem der vorangegangenen Ansprüche, wobei das modifizierte Serpin eine Mutation an P1 umfasst, wobei der P1-Rest gegebenenfalls zu R mutiert ist.

7. Modifiziertes Serpin nach einem der Ansprüche 1 bis 5, wobei das modifizierte Serpin einen R-Rest an Position P1 aufweist.

8. Modifiziertes Serpin nach einem der Ansprüche 1 bis 7, wobei die Mutationen in der RCL des modifizierten Serpins aus Mutationen an den Positionen P1' und P2; an den Positionen P1', P2 und P1; an den Positionen P1', P2 und P4 oder an den Positionen P1', P2, P4 und P1 bestehen.

9. Modifiziertes Serpin nach einem der Ansprüche 1 bis 8, wobei;
der P4-Rest in der RCL des modifizierten Serpins A ist, der P2-Rest K ist und der P1'-Rest K ist;
der P4-Rest in der RCL des modifizierten Serpins A ist, der P2-Rest R ist und der P1'-Rest H ist;
der P4-Rest in der RCL des modifizierten Serpins A ist, der P2-Rest K ist und der P1'-Rest H ist;
der P4-Rest in der RCL des modifizierten Serpins A ist, der P2-Rest R ist und der P1'-Rest Q ist;
der P4-Rest in der RCL des modifizierten Serpins A ist, der P2-Rest K ist und der P1'-Rest Q ist;
der P4-Rest in der RCL des modifizierten Serpins A ist, der P2-Rest K ist, der P1-Rest R und der P1'-Rest K ist;
der P4-Rest in der RCL des modifizierten Serpins A ist, der P2-Rest K ist, der P1-Rest R ist und der P1'-Rest Q ist; oder
der P4-Rest in der RCL des modifizierten Serpins A ist, der P2-Rest R ist, der P1-Rest R ist und der P1'-Rest Q ist;
der P4-Rest in der RCL des modifizierten Serpins K ist, der P2-Rest R ist und der P1'-Rest H ist,
der P4-Rest in der RCL des modifizierten Serpins F ist, der P2-Rest K ist und der P1'-Rest K ist;
der P4-Rest in der RCL des modifizierten Serpins F ist, der P2-Rest R ist und der P1'-Rest K ist; oder
der P4-Rest in der RCL des modifizierten Serpins S ist, der P2-Rest H ist und der P1'-Rest R ist.

10. Modifiziertes Serpin nach einem der vorangegangenen Ansprüche, das eine Aminosäuresequenz umfasst, die zumindest 70 % Sequenzidentität mit der Sequenz eines Wildtyp-Serpins aufweist.

11. Modifiziertes Serpin nach Anspruch 10, wobei das Wildtyp-Serpin aus der aus α₁-Antichymotrypsin (SERPINA3), C1-Esterasehemmer, α₂-Antiplasmin (SERPINF2), Antithrombin (ATIII) (SERPINC1), Heparin-Cofactor II (HCII) (SERPIND1), Protein-C-Hemmer (PCI) (SERPINA5) oder α₁₋Antitrypsin (α₁AT) (SERPINA1), Kallistatin (SERPINA4), Plasminogen-Aktivator-Hemmer (SERPINE1), Proteasenexin 1 (SERPINE2) und Protein Z-abhängigem Proteasehemmer (SERPINA10) bestehenden Gruppe ausgewählt ist.

12. Modifiziertes Serpin nach einem der Ansprüche 1 bis 11, das eine Aminosäuresequenz umfasst, die zumindest 70 % Sequenzidentität mit den Resten 25-418 von SEQ ID NO:12 aufweisen, wobei der P4-Rest in der RCL des modifizierten Serpins A ist, der P2-Rest K ist, der P1-Rest R ist und der P1'-Rest K ist.

13. Modifiziertes Serpin nach Anspruch 12, das Folgendes umfasst:
(i) die Aminosäuresequenz der Reste 25-418 von SEQ ID NO:12;
(ii) die Aminosäuresequenz der Reste 25-418 von SEQ ID NO:12 mit einer C-zu-S-Mutation an Rest 256;
(iii) die Aminosäuresequenz der Reste 25-418 von SEQ ID NO:12 mit einer E-zu-S-Mutation an Rest 25; oder
(iv) die Aminosäuresequenz der Reste 25-418 von SEQ ID NO:12 mit einer E-zu-S-Mutation an Rest 25 und eine C-zuS-Mutation an Rest 256.

14. Nucleinsäure, die für ein modifiziertes Serpin nach einem der Ansprüche 1 bis 13 kodiert; Vektor, der eine Nucleinsäure umfasst, die für ein modifiziertes Serpin nach einem der Ansprüche 1 bis 13 kodiert; oder rekombinante Zelle, die einen Vektor umfasst, der ein modifiziertes Serpin nach einem der Ansprüche 1 bis 13 exprimiert.

15. Pharmazeutische Zusammensetzung, die ein modifiziertes Serpin nach einem der Ansprüche 1 bis 13 und einen pharmazeutisch annehmbaren Exzipienten umfasst.

16. Modifiziertes Serpin nach einem der Ansprüche 1 bis 13 zur Verwendung bei der Behandlung des menschlichen oder tierischen Körpers.

17. Modifiziertes Serpin nach einem der Ansprüche 1 bis 13 zur Verwendung bei der Behandlung oder Prävention von Blutung oder zur Förderung von Hämostase in einem Individuum.

18. Modifiziertes Serpin zur Verwendung nach Anspruch 17, wobei das Individuum eine Blutungsstörung aufweist, wobei die Blutungsstörung gegebenenfalls Hämophilie ist.

19. Modifiziertes Serpin zur Verwendung nach Anspruch 17, wobei das Individuum ein Traumapatient ist.

## Revendications

1. Serpine modifiée comportant des mutations aux résidus P1' et P2 et facultativement P1 et/ou P4 dans la boucle centrale réactive (RCL) de celle-ci,
dans laquelle le résidu P1' est muté en Q, H, K ou R ; et le résidu P2 est muté en H, K ou R, et ;
dans laquelle lesdites mutations augmentent l'inhibition d'une ou de plusieurs protéases anticoagulantes sélectionnées parmi la protéine C activée et le complexe thrombine:thrombomoduline par rapport à l'inhibition d'une ou de plusieurs protéases procoagulantes sélectionnées parmi la thrombine, le fVIIa, le fXa, le fIXa et le fXIa.

2. Serpine modifiée selon la revendication 1, dans laquelle le résidu P1' est muté en K ou Q.

3. Serpine modifiée selon la revendication 1 ou la revendication 2, dans laquelle le résidu P2 est muté en K.

4. Serpine modifiée selon la revendication 1, dans laquelle les résidus P2 et P1' respectivement dans la serpine modifiée sont KK, RK, RH, KH, RQ ou KQ.

5. Serpine modifiée selon l'une quelconque des revendications précédentes, où la serpine modifiée comprend une mutation à P4, facultativement dans laquelle le résidu P4 est muté en F, S, R, V, C, W, K, G, L, H, T, Q ou A.

6. Serpine modifiée selon l'une quelconque des revendications précédentes, où la serpine modifiée comprend une mutation à P1, facultativement dans laquelle le résidu P1 est muté en R.

7. Serpine modifiée selon l'une quelconque des revendications 1 à 5, où la serpine modifiée comporte un résidu R à la position P1.

8. Serpine modifiée selon l'une quelconque des revendications 1 à 7, dans laquelle les mutations dans la RCL de la serpine modifiée consistent en des mutations aux positions P1' et P2 ; positions P1', P2 et P1 ; positions P1', P2 et P4 ; ou positions P1', P2, P4 et P1.

9. Serpine modifiée selon l'une quelconque des revendications 1 à 8, dans laquelle :
le résidu P4 dans la RCL de la serpine modifiée est A, le résidu P2 est K, et le résidu P1' est K ;
le résidu P4 dans la RCL de la serpine modifiée est A, le résidu P2 est R, et le résidu P1' est H ;
le résidu P4 dans la RCL de la serpine modifiée est A, le résidu P2 est K, et le résidu P1' est H ;
le résidu P4 dans la RCL de la serpine modifiée est A, le résidu P2 est R, et le résidu P1' est Q ;
le résidu P4 dans la RCL de la serpine modifiée est A, le résidu P2 est K, et le résidu P1' est Q ;
le résidu P4 dans la RCL de la serpine modifiée est A, le résidu P2 est K, le résidu P1 est R et le résidu P1' est K ;
le résidu P4 dans la RCL de la serpine modifiée est A, le résidu P2 est K, le résidu P1 est R et le résidu P1' est Q ; ou
le résidu P4 dans la RCL de la serpine modifiée est A, le résidu P2 est R, le résidu P1 est R et le résidu P1' est Q ;
le résidu P4 dans la RCL de la serpine modifiée est K, le résidu P2 est R et le résidu P1' est H ;
le résidu P4 dans la RCL de la serpine modifiée est F, le résidu P2 est K et le résidu P1' est K ;
le résidu P4 dans la RCL de la serpine modifiée est F, le résidu P2 est R et le résidu P1' est K ; ou
le résidu P4 dans la RCL de la serpine modifiée est S, le résidu P2 est H et le résidu P1' est R.

10. Serpine modifiée selon l'une quelconque des revendications précédentes qui comprend une séquence d'acides aminés qui présente au moins 70 % d'identité de séquence avec la séquence d'une serpine de type sauvage.

11. Serpine modifiée selon la revendication 10, dans laquelle la serpine de type sauvage est sélectionnée dans le groupe consistant en l'α₁-antichymotrypsine (SERPINA3), l'inhibiteur de la C1-estérase, l'α₂-antiplasmine (SERPINF2), l'antithrombine (ATIII) (SERPINC1), le cofacteur II de l'héparine (HCII) (SERPIND1), l'inhibiteur de la protéine C (PCI) (SERPINA5) ou l'α₁-antitrypsine (α₁AT) (SERPINA1), la kallistatine (SERPINA4), l'inhibiteur de l'activateur du plasminogène (SERPINE1), la protéase nexine 1 (SERPINE2) et l'inhibiteur de protéase dépendant de la protéine Z (SERPINA10).

12. Serpine modifiée selon l'une quelconque des revendications 1 à 11 comprenant une séquence d'acides aminés présentant au moins 70 % d'identité de séquence avec les résidus 25-418 de SEQ ID NO: 12, dans laquelle le résidu P4 dans la RCL de la serpine modifiée est A, le résidu P2 est K, le résidu P1 est R et le résidu P1' est K.

13. Serpine modifiée selon la revendication 12 comprenant :
(i) la séquence d'acides aminés des résidus 25-418 de SEQ ID NO: 12 ;
(ii) la séquence d'acides aminés des résidus 25-418 de SEQ ID NO: 12 avec une mutation de C à S au résidu 256 ;
(iii) la séquence d'acides aminés des résidus 25-418 de SEQ ID NO: 12 avec une mutation de E à S au résidu 25 ; ou
(iv) la séquence d'acides aminés des résidus 25-418 de SEQ ID NO: 12 avec une mutation de E à S au résidu 25 et une mutation de C à S au résidu 256.

14. Acide nucléique codant pour une serpine modifiée selon l'une quelconque des revendications 1 à 13, vecteur comprenant un acide nucléique codant pour une serpine modifiée selon l'une quelconque des revendications 1 à 13 ou cellule recombinante comprenant un vecteur qui exprime une serpine modifiée selon l'une quelconque des revendications 1 à 13.

15. Composition pharmaceutique comprenant une serpine modifiée selon l'une quelconque des revendications 1 à 13 et un excipient pharmaceutiquement acceptable.

16. Serpine modifiée selon l'une quelconque des revendications 1 à 13 destinée à être utilisée dans le traitement du corps humain ou animal.

17. Serpine modifiée selon l'une quelconque des revendications 1 à 13 destinée à être utilisée dans le traitement ou la prévention d'une hémorragie ou la promotion de l'hémostase chez un individu.

18. Serpine modifiée destinée à être utilisée selon la revendication 17, où l'individu présente un trouble hémorragique, facultativement où le trouble hémorragique est l'hémophilie.

19. Serpine modifiée destinée à être utilisée selon la revendication 17, où l'individu est un patient traumatisé.
